# EUROPEAN PATENT SPECIFICATION

(11) **EP 3 571 217 B1**
(45) Date of publication and mention of the grant of the patent: **21.06.2023**
(21) Application number: 18710940.0
(22) Date of filing: 17.01.2018
(51) Int. Cl.: C07K 14/32, C12N 15/09

(54) **METHODS AND COMPOSITIONS FOR OBTAINING NATURAL COMPETENCE IN BACILLUS HOST CELLS**
VERFAHREN UND ZUSAMMENSETZUNGEN ZUR GEWINNUNG VON NATÜRLICHER KOMPETENZ IN BACILLUS-WIRTSZELLEN
PROCÉDÉS ET COMPOSITIONS POUR OBTENIR UNE COMPÉTENCE NATURELLE DANS DES CELLULES HÔTES DE BACILLUS

(30) Priority: 23.01.2017 US 201762449496 P
(43) Date of publication of application: 27.11.2019
(73) Proprietor: Danisco US Inc., Palo Alto, California 94304 (US)
(72) Inventor: KOLKMAN, Marc Anton Bernhard, Palo Alto California 94304 (US); KOOPMAN, Frank Woute, Palo Alto California 94304 (US); LEEFLANG, Chris, Palo Alto California 94304 (US)
(74) Representative: Mewburn Ellis LLP
(86) International application number: PCT/US2018/013963
(87) International publication number: WO 2018/136459

(56) References cited:
- WO-A2-02/14490
- JAKOBS MAREIKE ET AL: "The two putative comS-homologs of the biotechnologically important Bacillus licheniformis do not contribute to competence development", APPLIED MICROBIOLOGY AND BIOTECHNOLOGY, SPRINGER, DE, vol. 99, no. 5, 19 December 2014 (2014-12-19), pages 2255-2266, XP035453349, ISSN: 0175-7598, DOI: 10.1007/S00253-014-6291-5 [retrieved on 2014-12-19] cited in the application
- JAKOBS MAREIKE ET AL: "What rendersBacilligenetically competent? A gaze beyond the model organism", APPLIED MICROBIOLOGY AND BIOTECHNOLOGY, SPRINGER, DE, vol. 99, no. 4, 31 December 2014 (2014-12-31), pages 1557-1570, XP035446294, ISSN: 0175-7598, DOI: 10.1007/S00253-014-6316-0 [retrieved on 2014-12-31]
- LIU LI ET AL: "Plasmid-amplified comS enhances genetic competence and suppresses sinR in Bacillus subtilis", JOURNAL OF BACTERIOLOGY, AMERICAN SOCIETY FOR MICROBIOLOGY, US, vol. 178, no. 17, 1 September 1996 (1996-09-01), pages 5144-5152, XP002488120, ISSN: 0021-9193
- L. W. HAMOEN: "Controlling competence in Bacillus subtilis: shared use of regulators", MICROBIOLOGY, vol. 149, no. 1, 1 January 2003 (2003-01-01), pages 9-17, XP055088717, ISSN: 1350-0872, DOI: 10.1099/mic.0.26003-0 cited in the application
- ASHIKAGA SAYAKA ET AL: "Natural genetic competence in Bacillus subtilis natto OK2", JOURNAL OF BACTERIOLOGY, AMERICAN SOCIETY FOR MICROBIOLOGY, US, vol. 182, no. 9, 1 May 2000 (2000-05-01), pages 2411-2415, XP002488123, ISSN: 0021-9193, DOI: 10.1128/JB.182.9.2411-2415.2000
- D'SOUZA CLETUS ET AL: "Identification of comS, a gene of the srfA operon that regulates the establishment of genetic competence in Bacillus subtilis", PROCEEDINGS OF THE NATIONAL ACADEMY OF SCIENCES, NATIONAL ACADEMY OF SCIENCES, US, vol. 91, no. 20, 1 January 1994 (1994-01-01), pages 9397-9401, XP002488122, ISSN: 0027-8424, DOI: 10.1073/PNAS.91.20.9397
- LAPIDUS ALLA ET AL: "Co-linear scaffold of the Bacillus licheniformis and Bacillus subtilis genomes and its use to compare their competence genes", FEMS MICROBIOLOGY LETTERS, WILEY-BLACKWELL PUBLISHING LTD, GB, vol. 209, no. 1, 19 March 2002 (2002-03-19), pages 23-30, XP002339776, ISSN: 0378-1097, DOI: 10.1111/J.1574-6968.2002.TB11104.X cited in the application
- MITSUO OGURA ET AL: "Mutational analysis of ComS: evidence for the interaction of ComS and MecA in the regulation of competence development in Bacillus subtilis", MOLECULAR MICROBIOLOGY., vol. 32, no. 4, 1 May 1999 (1999-05-01), pages 799-812, XP055469532, GB ISSN: 0950-382X, DOI: 10.1046/j.1365-2958.1999.01399.x

## Description

### CROSS-REFERENCE TO RELATED APPLICATIONS

This application claims the benefit of U.S. Provisional Patent Application Serial No. 62/449,496, filed January 23, 2017.

### FIELD OF THE INVENTION

The present disclosure is generally related to the fields of bacteriology, microbiology, genetics, molecular biology, enzymology and the like. Certain embodiments are directed to modified *Bacillus licheniformis* host cells comprising enhanced transformation efficiency and methods thereof for obtaining genetic competence in *Bacillus licheniformis* host cells.

### BACKGROUND OF THE INVENTION

The production of proteins *(e.g*., enzymes, antibodies, receptors, *etc.)* in microbial host cells is of particular interest in the biotechnological arts. Likewise, the optimization of microbial host cells for the production and secretion of one or more protein(s) of interest is of high relevance, particularly in the industrial biotechnology setting, wherein small improvements in protein yield are quite significant when the protein is produced in large industrial quantities.

Gram-positive bacteria such as *Bacillus subtilis, Bacillus licheniformis* and *Bacillus amyloliquefaciens* are frequently used as microbial factories for the production of industrial relevant proteins, due to their excellent fermentation properties and high yields (up to 25 grams per liter culture; Van Dijl and Hecker, 2013). For example, *B. subtilis* is well known for its production of α-amylases (Jensen *et al.,* 2000; Raul *et al.,* 2014) and proteases (Brode *et al.,* 1996) necessary for food, textile, laundry, medical instrument cleaning, pharmaceutical industries and the like (Westers *et al.,* 2004). Because these non-pathogenic Gram-positive bacteria produce proteins that completely lack toxic by-products (e.g., lipopolysaccharides; LPS, also known as endotoxins) they have obtained the "Qualified Presumption of Safety" (QPS) status of the European Food Safety Authority, and many of their products gained a "Generally Recognized As Safe" (GRAS) status from the US Food and Drug Administration (Olempska-Beer *et al.,* 2006; Earl *et al.,* 2008; Caspers *et al.,* 2010).

Genetic competence (or "natural genetic competence") is a physiological state in which exogenous DNA can be internalized, leading to a transformation event (Berka *et al.,* 2002). Natural competence/transformation is a distinct process from "artificial transformation", which involves methods such as electroporation, protoplasts, heat shock or CaCl₂ treatment. Natural competence has been observed in both Gram-positive and Gram-negative bacterial species (Dubnau, 1999), wherein the process requires more than a dozen proteins whose expression is precisely choreographed to the needs of each organism. Thus, in terms of practical utility, transformation via natural competence is an extremely useful tool for constructing genetically modified bacterial strains (*e.g*., constructing genetically modified *Bacillus* host cells, constructing *Bacillus* mutant libraries for protein engineering and the like). Although transformation of certain bacterial species with plasmids, chromosomal DNA and the like may be achieved via "artificial transformation" means, the introduction of DNA by "natural competence" offers clear advantages of simplicity, convenience, speed, and efficiency.

In *Bacillus subtilis,* approximately 5-10% of the cells in a population will differentiate to a competent state (termed the "K-state") by means of a process that involves quorum-sensing, signal transduction, and a cascade of gene expression (Avery, 2005). At least 50 genes are known to be involved directly in competence, and as many as 165 genes are regulated (directly or indirectly) by the central transcription factor ComK (Berka *et al.,* 2002). For example, the competence cascade/process in *Bacillus subtilis* consists of two regulatory modules punctuated by a molecular switch that involves ComS binding to the adaptor molecule MecA, thereby interfering with degradation of the transcription factor ComK by the CIpC/CIpP protease (Turgay *et al.,* 1998).

However, much less is known about natural competence in the closely related species *Bacillus licheniformis.* For example, it is known in the art that most *B*. *licheniformis* isolates do not manifest natural competence, but the reasons for the apparent lack of a competent state in *Bacillus licheniformis* are generally unknown or contradictory. Furthermore, as stated briefly above, *Bacillus licheniformis* is a *Bacillus* species host cell of high industrial importance, and as such, the ability to modify and engineer *B*. *licheniformis* host cells via natural competence (natural transformation) is highly desirable for construction of new and improved *Bacillus licheniformis* production strains.

Thus, certain embodiments of the present disclosure are related to the highly desirable and unmet needs for obtaining natural competence in *Bacillus spp.* host cells (*e.g., B*. *licheniformis)* host cells, increased transformation efficiency thereof and methods thereof.

### SUMMARY OF THE INVENTION

Certain embodiments of the instant disclosure are directed to modified *Bacillus licheniformis* host cells comprising enhanced transformation and methods thereof for obtaining genetic competence in *Bacillus licheniformis* host cells. Thus, in certain embodiments, the disclosure is directed to a method for enhancing transformation efficiency of a *Bacillus licheniformis* host cell comprising (a) introducing into a parental *Bacillus licheniformis* host cell at least one copy of an exogenous nucleic acid construct comprising a promoter region operably linked to a polynucleotide encoding a ComS1 polypeptide, wherein the ComS1 polypeptide comprises an amino acid sequence comprising at least 90% sequence identity to SEQ ID NO: 2, and (b) isolating a daughter *Bacillus licheniformis* host cell comprising the polynucleotide encoding the ComS1 polypeptide.

In another embodiment, the polynucleotide encoding the ComS1 polypeptide comprises at least 90% sequence identity to nucleotide sequence of SEQ ID NO: 1 or SEQ ID NO: 21. In another embodiment, the *Bacillus licheniformis* host cell further comprises at least one copy of an exogenously introduced nucleic acid construct comprising a promoter region operably linked to a polynucleotide encoding a ComK polypeptide. In certain embodiments, the ComK polypeptide comprises an amino acid sequence comprising 90% sequence identity to SEQ ID NO: 17 or SEQ ID NO: 18.

In another embodiment, the competent *Bacillus licheniformis* host cell is transformed with an exogenously introduced polynucleotide encoding a protein of interest (POI). In other embodiments, the POI is selected from the group consisting of acetyl esterases, aminopeptidases, amylases, arabinases, arabinofuranosidases, carbonic anhydrases, carboxypeptidases, catalases, cellulases, chitinases, chymosins, cutinases, deoxyribonucleases, epimerases, esterases, α-galactosidases, β-galactosidases, α-glucanases, glucan lysases, endo-β-glucanases, glucoamylases, glucose oxidases, α-glucosidases, β-glucosidases, glucuronidases, glycosyl hydrolases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, peroxidases, phenoloxidases, phytases, polygalacturonases, proteases, peptidases, rhamno-galacturonases, ribonucleases, transferases, transport proteins, transglutaminases, xylanases, hexose oxidases, and combinations thereof.

Certain other embodiments are directed to the competent *Bacillus licheniformis* host cell obtained from the methods described above.

In other embodiments, the disclosure is directed to a method for obtaining a *Bacillus licheniformis* transformant comprising (a) providing a *Bacillus licheniformis* host cell in which transformation efficiency has been enhanced by introducing into the host cell at least one copy of a nucleic acid construct comprising a promoter region operably linked to a polynucleotide encoding a ComS1 polypeptide, wherein the ComS1 polypeptide comprises an amino acid sequence comprising at least 90% sequence identity to SEQ ID NO: 2, (b) transforming an exogenous polynucleotide into a *Bacillus licheniformis* host cell provided in step (a), and (c) isolating a transformant of the *Bacillus licheniformis* host cell comprising the exogenously introduced polynucleotide. In certain embodiments, the method for enhancing transformation efficiency, or for obtaining, the parental *Bacillus licheniformis* host cell, does not natively comprise a ComS1 polypeptide comprising an amino acid sequence comprising at least 90% sequence identity to SEQ ID NO: 2. In other embodiments, the polynucleotide encoding the ComS1 polypeptide comprises at least 90% sequence identity to nucleotide sequence of SEQ ID NO: 1 or SEQ ID NO: 21. In certain other embodiments, the *Bacillus licheniformis* host cell further comprises at least one copy of an exogenously introduced nucleic acid construct comprising a promoter region operably linked to a polynucleotide encoding a ComK polypeptide. In yet other embodiments, the ComK polypeptide comprises an amino acid sequence comprising 90% sequence identity to SEQ ID NO: 17 or SEQ ID NO: 18. In other embodiments, the exogenous polynucleotide of step (b) encodes a protein of interest (POI) selected from the group consisting of acetyl esterases, aminopeptidases, amylases, arabinases, arabinofuranosidases, carbonic anhydrases, carboxypeptidases, catalases, cellulases, chitinases, chymosins, cutinases, deoxyribonucleases, epimerases, esterases, α-galactosidases, β-galactosidases, α-glucanases, glucan lysases, endo-β-glucanases, glucoamylases, glucose oxidases, α-glucosidases, β-glucosidases, glucuronidases, glycosyl hydrolases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, peroxidases, phenoloxidases, phytases, polygalacturonases, proteases, peptidases, rhamno-galacturonases, ribonucleases, transferases, transport proteins, transglutaminases, xylanases, hexose oxidases, and combinations thereof.

Certain other embodiments are directed to the transformed *Bacillus licheniformis* host cell obtained from the methods described above.

In another embodiment, the disclosure is directed to a method for enhancing transformation efficiency of a *Bacillus licheniformis* (daughter) host cell derived from a parental *Bacillus licheniformis* host cell, wherein the parental *Bacillus licheniformis* host cell comprises a native gene encoding a ComS2 polypeptide comprising 90% sequence identity to the ComS2 polypeptide of SEQ ID NO: 4, the method comprising: (a) identifying a parental *Bacillus licheniformis* host cell which comprises a native gene encoding a ComS2 polypeptide comprising 90% sequence identity to the ComS2 polypeptide of SEQ ID NO: 4, (b) deleting or disrupting the native ComS2 gene identified in the parental *Bacillus licheniformis* host cell of step (a), wherein the deletion or disruption of the native ComS2 gene enhances the transformation efficiency of the *Bacillus licheniformis* daughter host cell derived therefrom, and (c) isolating the daughter *Bacillus licheniformis* host cell of step (b). In certain embodiments, the *Bacillus licheniformis* daughter host cell isolated in step (c) is further modified by introducing at least one copy of an exogenously introduced nucleic acid construct comprising a promoter region operably linked to a polynucleotide sequence encoding a ComS1 polypeptide, wherein the ComS1 polypeptide comprises at least 90% sequence identity to SEQ ID NO: 2. In other embodiments, the polynucleotide encoding the ComS1 polypeptide comprises at least 90% sequence identity to nucleotide sequence of SEQ ID NO: 1 or SEQ ID NO: 21. In another embodiment, the *Bacillus licheniformis* host cell further comprises at least one copy of an exogenously introduced nucleic acid construct comprising a promoter region operably linked to a polynucleotide encoding a ComK polypeptide. In certain embodiments, the ComK polypeptide comprises an amino acid sequence comprising 90% sequence identity to SEQ ID NO: 17 or SEQ ID NO: 18. In yet other embodiments, the competent *Bacillus licheniformis* host cell is transformed with an exogenously introduced polynucleotide encoding a protein of interest (POI).
In certain embodiments, the POI is selected from the group consisting of acetyl esterases, aminopeptidases, amylases, arabinases, arabinofuranosidases, carbonic anhydrases, carboxypeptidases, catalases, cellulases, chitinases, chymosins, cutinases, deoxyribonucleases, epimerases, esterases, α-galactosidases, β-galactosidases, α-glucanases, glucan lysases, endo-β-glucanases, glucoamylases, glucose oxidases, α-glucosidases, β-glucosidases, glucuronidases, glycosyl hydrolases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, peroxidases, phenoloxidases, phytases, polygalacturonases, proteases, peptidases, rhamno-galacturonases, ribonucleases, transferases, transport proteins, transglutaminases, xylanases, hexose oxidases, and combinations thereof.

In other embodiments, the disclosure is directed to the competent *Bacillus licheniformis* host cell obtained from the method described above.

In other embodiments, the disclosure is directed to a competent *Bacillus licheniformis* host cell comprising at least one copy of an exogenously introduced nucleic acid construct comprising a promoter region operably linked to a polynucleotide sequence encoding a ComS1 polypeptide, wherein the ComS1 polypeptide comprises at least 90% sequence identity to SEQ ID NO: 2In another embodiment, the competent host cell is transformed with an exogenous polynucleotide encoding a protein of interest (POI). In yet other embodiments, the polynucleotide encoding the ComS1 polypeptide comprises at least 90% sequence identity to nucleotide sequence of SEQ ID NO: 1 or SEQ ID NO: 21. In another embodiment, the competent host cell further comprises at least one copy of an exogenously introduced nucleic acid construct comprising a promoter region operably linked to a polynucleotide encoding a ComK polypeptide. In certain embodiments, the ComK polypeptide comprises an amino acid sequence comprising 90% sequence identity to SEQ ID NO: 17 or SEQ ID NO: 18. Also described is a promoter region operably linked to the polynucleotide sequence encoding the ComS1 polypeptide is a constitutive promoter or an inducible promoter. In a certain disclosure, the exogenously introduced nucleic acid encoding a POI is comprised in an expression vector. In another certain disclosure, the exogenously introduced nucleic acid construct encoding a POI is integrated into the chromosome of the host cell. In still other disclosures, the exogenously introduced nucleic acid construct comprising a promoter region operably linked to a polynucleotide encoding a
comK polypeptide is comprised in an expression vector. In certain disclosures, the exogenously introduced nucleic acid encoding a comK polypeptide is integrated into the chromosome of the host cell.

Certain other disclosures are directed to a protein of interest produced by the competent host cell of the disclosure.

In another embodiment, the disclosure is directed to a transformed *Bacillus licheniformis* host cell made competent by at least one copy of an exogenously introduced nucleic acid construct comprising a promoter region operably linked to a polynucleotide encoding a ComS1 polypeptide comprising 90% sequence identity to SEQ ID NO: 2, wherein the *Bacillus licheniformis* host cell is transformed with a polynucleotide encoding a protein of interest (POI). In certain embodiments, the transformed host cell comprises a polynucleotide encoding the ComS1 polypeptide, wherein the polynucleotide comprises at least 90% sequence identity to nucleotide sequence of SEQ ID NO: 1 or SEQ ID NO: 21. In other embodiments, the transformed host cell further comprises at least one copy of an exogenously introduced nucleic acid construct comprising a promoter region operably linked to a polynucleotide encoding a ComK polypeptide. In certain embodiments, the ComK polypeptide comprises an amino acid sequence comprising 90% sequence identity to SEQ ID NO: 17 or SEQ ID NO: 18. In another disclosure, the promoter region operably linked to the polynucleotide sequence encoding the ComS1 polypeptide is a constitutive promoter or an inducible promoter. In still another disclosure, the polynucleotide encoding a POI is comprised in an expression vector , or the polynucleotide encoding the POI is an expression cassette integrated into the chromosome of the host cell.

Certain other embodiments of the disclosure are directed to one or more proteins of interest produced by such transformed *Bacillus licheniformis* host cells.

### BRIEF DESCRIPTION OF DRAWINGS

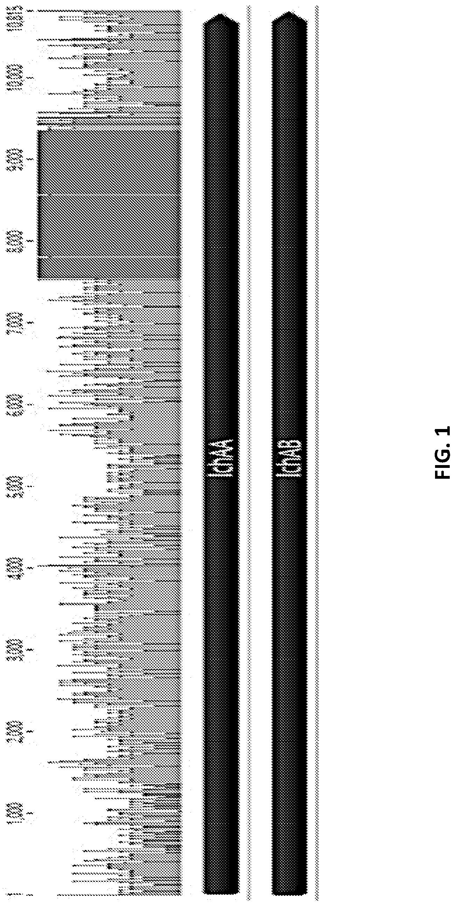

**Figure 1** is a nucleic acid sequence alignment plot comparing the *B*. *licheniformis* (parental) strain Bra7 "IchAA" gene and "IchAB" gene. The numbers presented indicate the base pairs of the gene alignment. The green bars represent a 100% identity of the base pairs between the two genes (*i.e*., IchAA and IchAB). Green/Brown bars represent at least 30%, but lower than 100%, identity of the base pairs between the two genes. Red indicates less than 30% identity of the base pairs between the two genes.

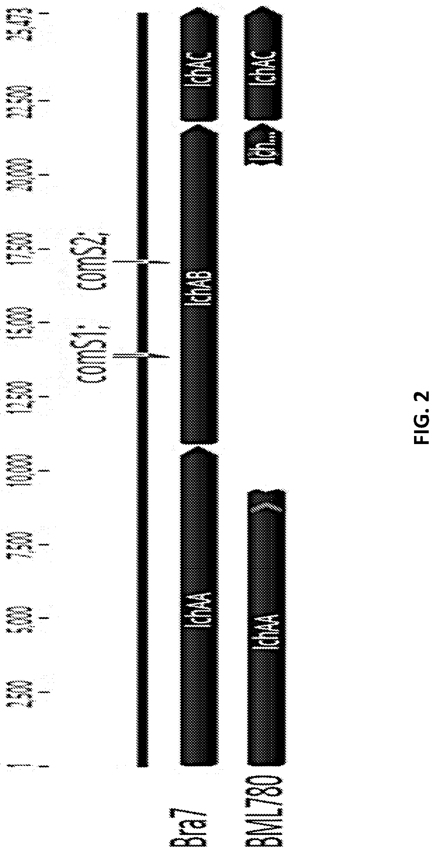

**Figure 2** shows a physical map of the homology of the *lch* locus of the (parental) *B*. *licheniformis* Bra7 strain host cell (FIG. 2, upper locus) compared to the physical map of the *Ich* locus of a (daughter) *B*. *licheniformis* host cell (FIG. 2; lower locus) (*i*.*e*., a daughter cell derived from the parental *B*. *licheniformis* Bra7 strain), wherein the numbers presented indicate the base pairs of the locus. The *comS1* and *comS2* annotated genes are located within the *lchAB* gene present in the parental *B*. *licheniformis* host cell (strain Bra7), but are absent in the (daughter) *B*. *licheniformis* host cells derived from the (parental) *B*. *licheniformis* Bra7 strain.

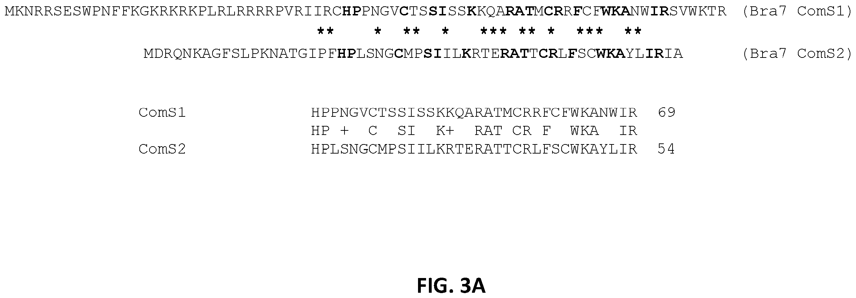

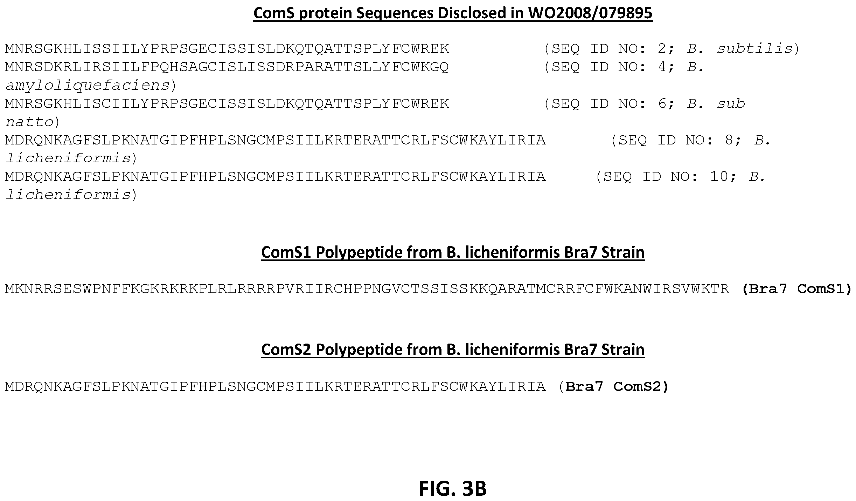

**Figure 3** shows the amino acid sequences of certain ComS polypeptides. **FIG. 3A** shows the amino acid sequences of *B*. *licheniformis* Bra7 strain ComS1 and ComS2 polypeptides, which share approximately 50% sequence identity between residues 36-69 of ComS1 and residues 21-54 of ComS2. **FIG. 3B** shows an amino acid sequence alignment of ComS polypeptides from *B*. *subtilis* (*i.e.,* FIG. 3B, SEQ ID NO: 2), *B*. *amyloliquefaciens* (*i.e.,* FIG. 3B, SEQ ID NO: 4), *B*. *subtilis natto* (*i.e.*, FIG. 3B, SEQ ID NO: 6) and *B*. *licheniformis* (*i.e.,* FIG. 3B, SEQ ID NO: 8 and SEQ ID NO: 10) as disclosed in PCT Publication No. WO2008/079895 relative to the *B*. *licheniformis* Bra7 strain ComS1 polypeptide (*i.e*., SEQ ID NO: 2 of instant disclosure) and *B*. *licheniformis* Bra7 strain ComS2 polypeptide (*i.e*., SEQ ID NO: 4 of instant disclosure) As presented in FIG. 3B, the *B*. *licheniformis* ComS1 polypeptide from *B*. *licheniformis* Bra7 strain (SEQ ID NO: 2 of instant disclosure) shares approximately 40-50% sequence identity with the ComS polypeptides disclosed in PCT Publication No. WO2008/079895 and the ComS2 polypeptide from *B*. *licheniformis* Bra7 strain (SEQ ID NO: 4 of instant disclosure) shares approximately 40-50% sequence identity with the *B*. *subtilis* (SEQ ID NO: 2), *B*. *amyloliquefaciens* (SEQ ID NO: 4) and *B*. *subtilis natto* (SEQ ID NO: 6) ComS polypeptides disclosed in PCT Publication No. WO2008/079895. More particularly, FIG. 3B shows that the ComS2 polypeptide from *B*. *licheniformis* Bra7 strain (SEQ ID NO: 4 of instant disclosure) shares 100% sequence identity to the *B*. *licheniformis* ComS polypeptides (SEQ ID NO: 8 and SEQ ID NO: 10) disclosed in PCT Publication No. WO2008/079895.

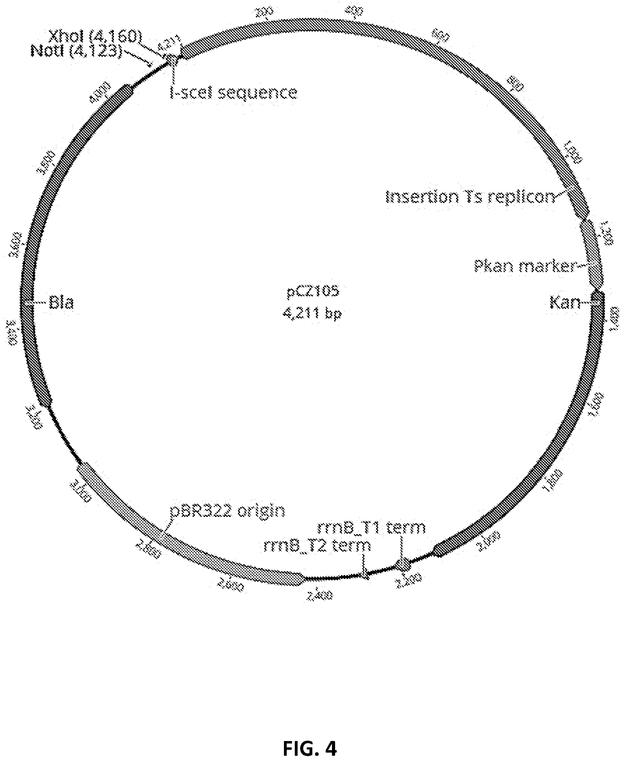

**Figure 4** shows a plasmid map of vector pCZ105, containing various restriction enzyme (RE) sites, amongst which are the RE sites *XhoI* and *NotI.* Also comprised in plasmid vector pCZ105 is a pE194 temperature sensitive replicon (Ts replicon), a kanamycin coding sequence (Kan), a kanamycin promoter (pKan marker), a ribosomal terminator sequence (Term rrnB), a β-lactamase ("bla") gene, an *I-Sce* site and a pBR322 origin of replication.

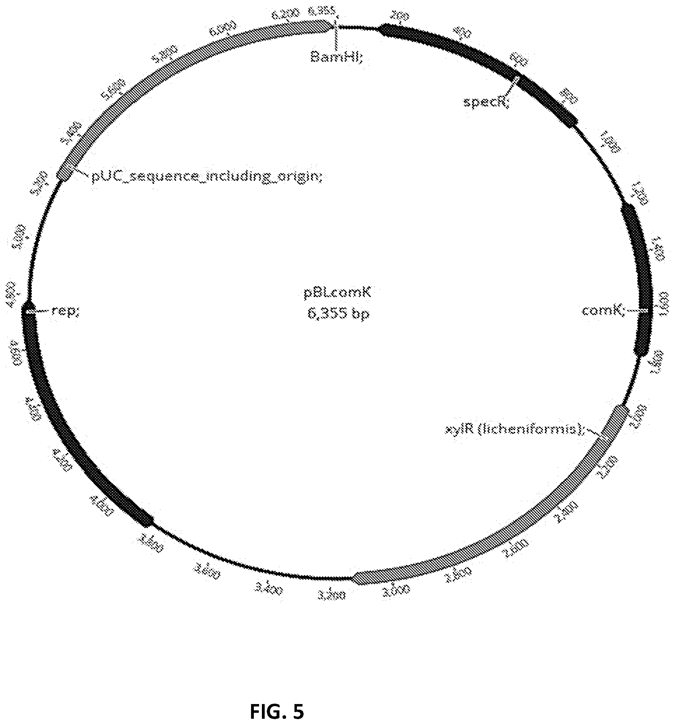

**Figure 5** shows a plasmid map of vector pBLcomK. Plasmid pBLcomK includes DNA sequences encoding the pBR322 origin of replication, the *Enterococcus faecalis* Spectinomycin resistance (Spec^{r}) gene *spc* (also called aad9), the *B*. *subtilis* (*natto*) plasmid pTA1060 *rep* gene for replication in *Bacilli,* the *B*. *licheniformis* comK gene controlled by the *B*. *subtilis xyl*A promoter, and the *B*. *subtilis xyl*R gene.

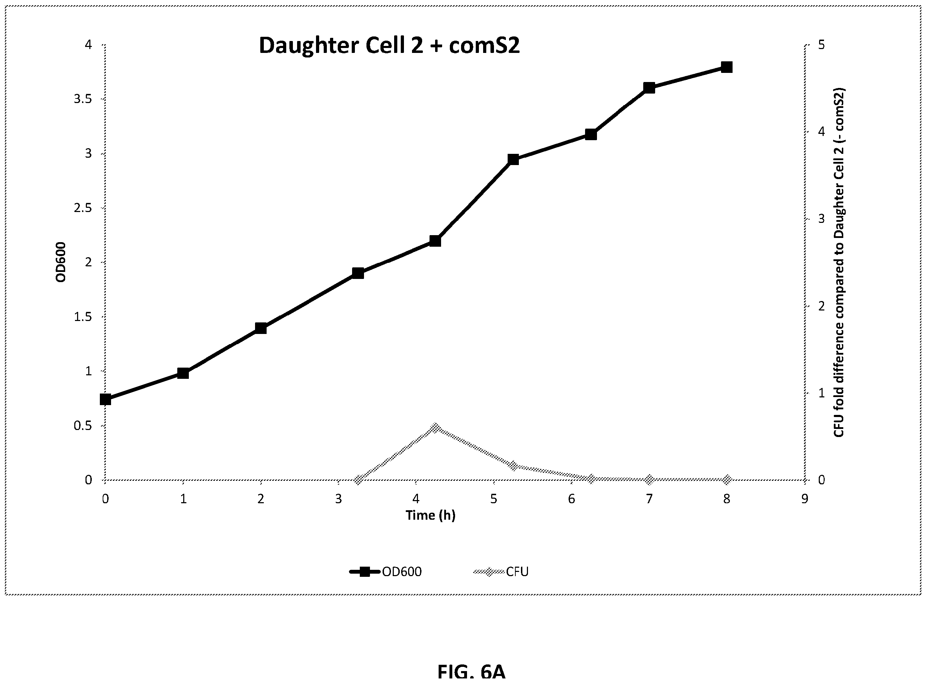

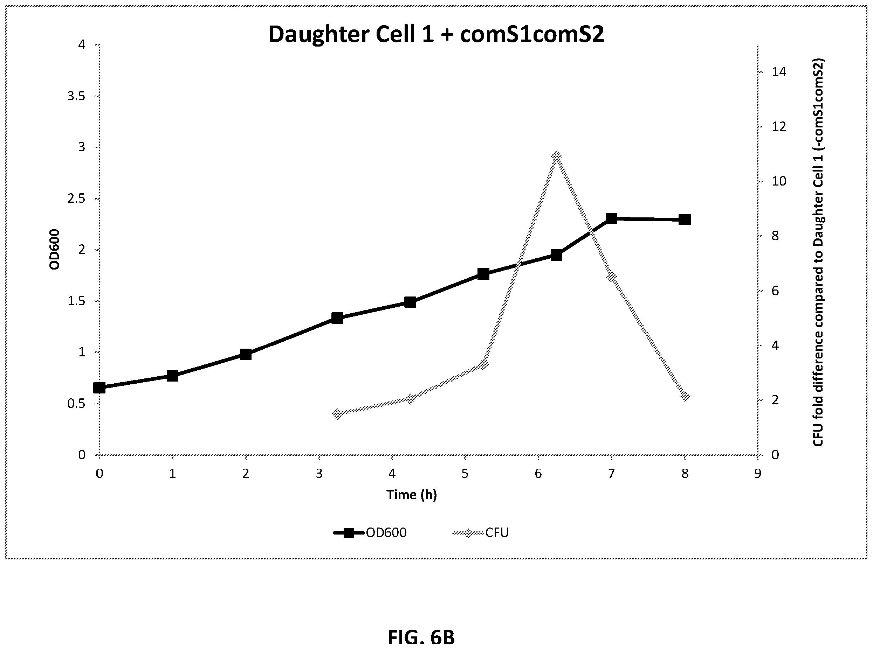

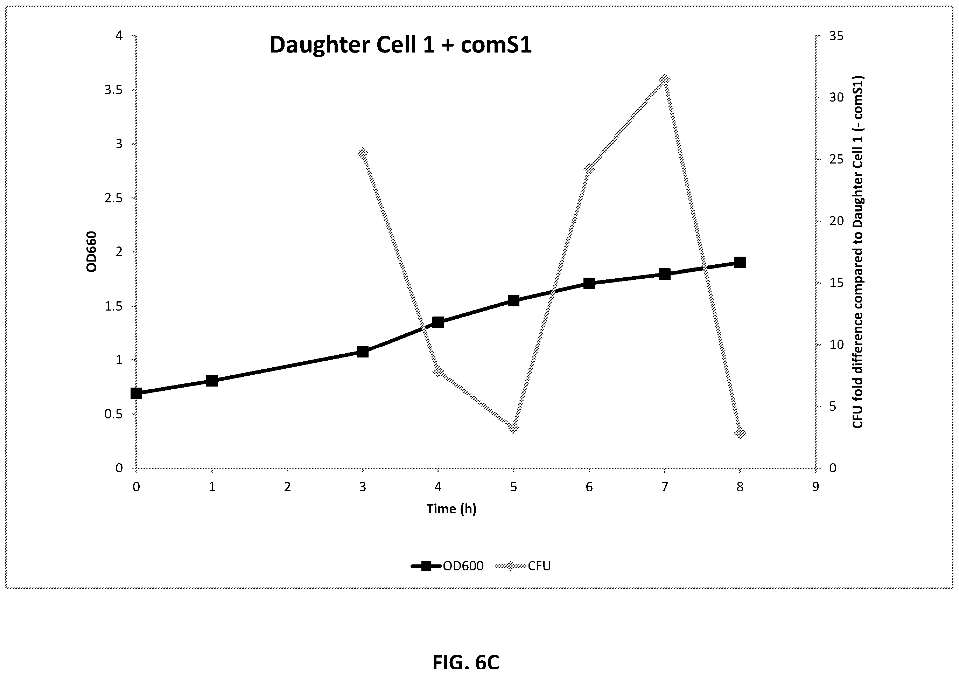

**Figure 6** shows the transformation efficiency and OD₆₀₀ of *B*. *licheniformis* host cells cultured and transformed during a competence assay. The black line indicates the OD₆₀₀, wherein the results presented are an average of duplicate experiments. **FIG. 6A-6C** show the OD₆₀₀ and transformation efficiency of *B*. *licheniformis* daughter cells derived from the parental *B. licheniformis* (Bra7 strain) host cell. More particularly, the B. *licheniformis Daughter Cell 2* presented in **FIG. 6A** was transformed with the *comS2* construct (*i.e*., Construct #2; Example 1), wherein the grey line indicates the CFU fold difference compared to the same *B*. *licheniformis Daughter Cell* 2 which has not been transformed with the *comS2* construct (Construct #2). The *B*. *licheniformis Daughter Cell* 1 presented in **FIG. 6B** was transformed with the *comS1comS2* construct (*i.e*., Construct #3; Example 1), wherein the grey line indicates the CFU fold difference compared to the same *B*. *licheniformis Daughter Cell 1* which has not been transformed with the *comS1comS2* construct (Construct # 3). The *B*. *licheniformis Daughter Cell 1* presented in **FIG. 6C** was transformed with the *comS1* construct (*i.e*., Construct # 1; Example 1), wherein the grey line indicates the CFU fold difference compared to the same *B. licheniformis Daughter Cell 1* which has not been transformed with the *comS1* construct (Construct # 3).

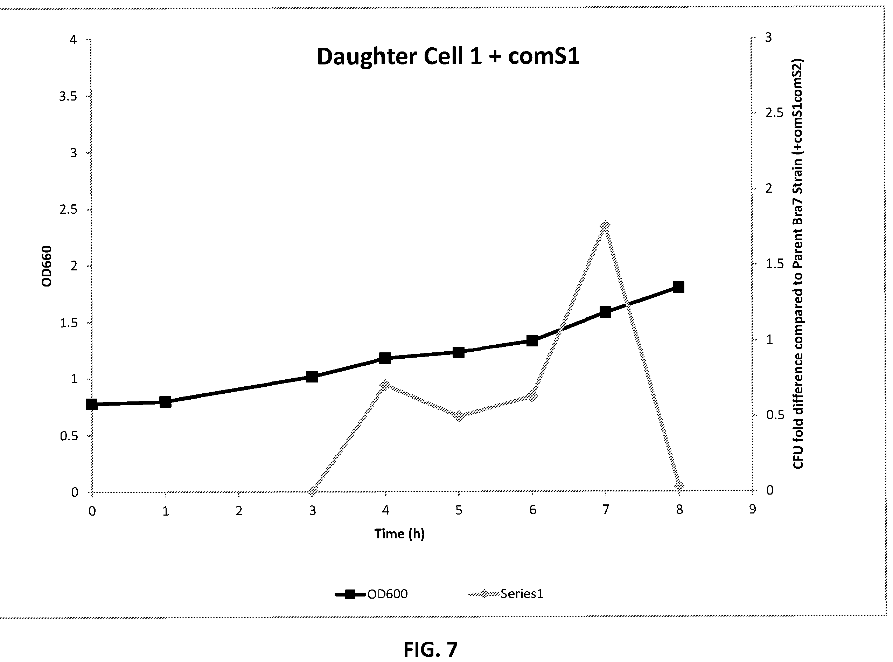

**Figure 7** shows the transformation efficiency and OD₆₀₀ of the *B*. *licheniformis Daughter Cell 1* transformed with the *comS1* construct (Construct # 1), wherein the grey line indicates the CFU fold difference compared vis-à-vis to the parental *B*. *licheniformis* (Bra7 strain) host cell which natively comprises both a *comS1* gene (SEQ ID NO: 21) encoding a ComS1 polypeptide (SEQ ID NO: 2) and a *comS*2 gene (SEQ ID NO: 22) encoding an ComS2 polypeptide (SEQ ID NO: 4).

### BRIEF DESCRIPTION OF THE BIOLOGICAL SEQUENCES

SEQ ID NO: 1 is a nucleic acid sequence encoding a *Bacillus licheniformis* ComS1 polypeptide of SEQ ID NO: 2.
SEQ ID NO: 2 is the amino acid sequence of a *Bacillus licheniformis* ComS1 polypeptide.
SEQ ID NO: 3 is a nucleic acid sequence encoding a *Bacillus licheniformis* ComS2 polypeptide of SEQ ID NO: 4.
SEQ ID NO: 4 is the amino acid sequence of a *Bacillus licheniformis* ComS2 polypeptide.
SEQ ID NO: 5 is a nucleic acid sequence comprising a promoter region. More specifically, the nucleic acid sequence of SEQ ID NO: 5 comprises a promoter and a 5' UTR, which promoter region can be operably linked to a polynucleotide sequence encoding a ComS polypeptide of the disclosure.
SEQ ID NO: 6 is a nucleic acid sequence comprising a 3' terminator sequence, which terminator sequence can be operably linked to a polynucleotide sequence encoding a ComS polypeptide of the disclosure.
SEQ ID NO: 7 is a nucleic acid sequence comprising ribosomal binding site (RBS).
SEQ ID NO: 8 is a primer sequence.
SEQ ID NO: 9 is a primer sequence.
SEQ ID NO: 10 is a primer sequence.
SEQ ID NO: 11 is a primer sequence.
SEQ ID NO: 12 is a primer sequence.
SEQ ID NO: 13 is a primer sequence.
SEQ ID NO: 14 is a primer sequence.
SEQ ID NO: 15 is a primer sequence.
SEQ ID NO: 16 is a primer sequence.
SEQ ID NO: 17 is the amino acid sequence of a *B. licheniformis* ComK polypeptide.
SEQ ID NO: 18 is the amino acid sequence of a *B. licheniformis* ComK polypeptide.
SEQ ID NO: 19 is a nucleic acid sequence with homology to upstream (5') regions of the parental *B. licheniformis* (host cell) *amyL* locus.
SEQ ID NO: 20 is a nucleic acid sequence with homology to downstream (3') regions of the parental *B. licheniformis* (host cell) *amyL* locus.
SEQ ID NO: 21 is a nucleic acid sequence encoding a *Bacillus licheniformis* ComS1 polypeptide of SEQ ID NO: 2.
SEQ ID NO: 22 is a nucleic acid sequence encoding a *Bacillus licheniformis* ComS2 polypeptide of SEQ ID NO: 4

### DETAILED DESCRIPTION

In certain embodiments, the present disclosure is directed to methods and compositions for increasing, enhancing and/or obtaining transformational efficiency in a parental *Bacillus licheniformis* host cell. Thus, certain embodiments of the disclosure are related to competent (daughter) *Bacillus licheniformis* host cells derived from non-competent or poorly competent (parental) *B. licheniformis* host cells. In certain other embodiments, a non-competent parental *Bacillus licheniformis* host cell is made competent by the introduction of at least one copy of an exogenously introduced polynucleotide encoding a *B. licheniformis* ComS1 polypeptide. In another embodiment, the at least one copy of the exogenously introduced polynucleotide encoding a *B. licheniformis* ComS1 polypeptide encodes a ComS1 polypeptide comprising at least 90% sequence identity to the *B*. *licheniformis ComS1* polypeptide of SEQ ID NO: 2. In other embodiments, a competent *B*. *licheniformis* host cell is transformed with a polynucleotide encoding a protein of interest (POI). In certain embodiments, a polynucleotide encoding a ComS1 polypeptide comprises a nucleotide sequence of SEQ ID NO: 1 or SEQ ID NO: 21, wherein nucleotide sequences of SEQ ID NO: 1 and SEQ ID NO: 21 differ only at the first 5' nucleotide, wherein the first 5' nucleotide of SEQ ID NO: 1 is an adenine (A) and the first 5' nucleotide of SEQ ID NO: 21 is a thymine (T). In certain embodiments, a polynucleotide encoding a ComS2 polypeptide comprises a nucleotide sequence of SEQ ID NO: 3 or SEQ ID NO: 22, wherein nucleotide sequences of SEQ ID NO: 3 and SEQ ID NO: 22 differ only at the second to last 3' nucleotide, wherein the second to last 3' nucleotide of SEQ ID NO: 3 is an adenine (A) and the second to last 3' nucleotide of nucleotide of SEQ ID NO: 22 is a guanine (G).

### I. Definitions

In view of the modified *Bacillus licheniformis* host cells, competent host cells thereof, transformed host cells thereof and methods thereof as described herein, the following terms and phrases are defined. Terms not defined herein should be accorded their ordinary meaning as used in the art.

Unless defined otherwise, all technical and scientific terms used herein have the same meaning as commonly understood by one of ordinary skill in the art to which the present compositions and methods apply. Although any methods and materials similar or equivalent to those described herein can also be used in the practice or testing of the present compositions and methods, representative illustrative methods and materials are now described.

It is further noted that the claims may be drafted to exclude any optional element. As such, this statement is intended to serve as antecedent basis for use of such exclusive terminology as "solely," "only", "excluding", "not including" and the like, in connection with the recitation of claim elements, or use of a "negative" limitation or proviso thereof.

As will be apparent to those of skill in the art upon reading this disclosure, each of the individual embodiments described and illustrated herein has discrete components and features which may be readily separated from or combined with the features of any of the other several embodiments without departing from the scope or spirit of the present compositions and methods described herein. Any recited method can be carried out in the order of events recited or in any other order which is logically possible.

As defined herein, the terms **"competence"** and **"natural competence"** are used interchangeably, and refer to a natural physiological state in which exogenous (extracellular) DNA can be taken up and internalized into a *Bacillus licheniformis* host cell, leading to a "transformation" event. In contrast, the term "artificial competence" as used herein refers to an artificial transformation event involving methods/techniques such as electroporation, protoplasts, heat shock, or CaCl₂ treatment and the like.

As defined herein, terms such as **"competence mechanism"** and **"competence cascade"** are used interchangeably and refer to a cellular differentiation process that converts *Bacillus* cells into naturally transformable cells *(i.e., via* natural competence) that can take up and incorporate exogenous (extracellular) DNA using specific transport proteins encoded by the late competence genes comprising the comC, comE, comF, and comG operons.

As defined herein, the terms **"non-competent"** cell, **"poorly competent"** cell and **"poorly transformable"** cell are used interchangeably, and these terms mean that the number of transformants per microgram of DNA is less than twice the spontaneous mutation frequency when using the methods for competence-mediated transformation in *Bacillus subtilis* or *Bacillus licheniformis* as described previously (Anagnostopoulos and Spizizen, 1961; Thome and Skill, 1966).

As defined herein, a *Bacillus licheniformis* **strain Bra7** (or **Bra7 strain)** is a *B*. *licheniformis* host cell developed/derived from a wild-type *B. licheniformis* parent strain using classical genetic improvements methods. Although certain embodiments and descriptions of the present disclosure are related to *B*. *licheniformis* strain Bra7, the compositions and methods of the instant disclosure are not limited to a specific *Bacillus* species. More particularly, although certain embodiments and descriptions of the present disclosure are related to *B. licheniformis* strain Bra7, the compositions and methods of the instant disclosure are not limited to a specific strain of *Bacillus licheniformis* host cells. For example, as disclosed herein, the introduction of a nucleic acid construct (comprising a promoter region operably linked to a polynucleotide encoding a ComS1 polypeptide comprising 90% sequence identity to SEQ ID NO: 2) into a non-competent (poorly transformable) *Bacillus licheniformis* host cell is used to obtain competent *Bacillus* host cells thereof. Thus, as presented in the Examples herein, introducing a nucleic acid construct encoding a ComS1 polypeptide into a non-competent *Bacillus licheniformis* host cell *(i.e*., which host cell does not natively comprise a gene encoding a ComS1 polypeptide) renders such *Bacillus licheniformis* host cell competent. In addition, as described herein, introducing a nucleic acid construct encoding a ComS1 polypeptide into a poorly competent or low competency *Bacillus licheniformis* host cell which comprises a native gene encoding a ComS1 polypeptide, significantly increases (enhances) the competency (transformability) of such *Bacillus licheniformis* host cells comprising a native gene encoding ComS1.

As used herein, the term ***"Daughter Cell* 1"** or in the abbreviated form ***"DC1",*** specifically refers to a *B. licheniformis* (daughter) cell derived from a parental *B. licheniformis* Bra7 (strain) host cell. More particularly, as used herein, the *B. licheniformis "Daughter Cell 1"* or *"DC1"* derived from the *B. licheniformis* Bra7 parent comprises a five (5) gene deletion (Δcat, ΔamyL, Δspo, ΔaprL, ΔendoGluC) as described in PCT International Publication No. WO2008/024372.

As used herein, the term ***"Daughter Cell 2*"** or in the abbreviated form ***"DC2"**,* specifically refers to a *B. licheniformis* (daughter) cell derived from a parental *B. licheniformis* Bra7 (strain) host cell. More particularly, as used herein, the *B. licheniformis "Daughter Cell 2"*or *"DC2"* derived from the *B. licheniformis* Bra7 parent comprises the same five (5) gene deletions as described for *"Daughter Cell 1"* above (*i.e*., Δcat, ΔamyL, Δspo, ΔaprL, ΔendoGluC; *see*, PCT International Publication No. WO2008/024372) and further comprises a modified *rpoC* gene encoding a rpoC polypeptide comprising an aspartic acid (D) to glycine (G) substitution at residue position 796 of the rpoC polypeptide, as generally described in PCT International Application No. PCT/US2016/059078, filed October 27, 2016.

As used herein, the term **"ComK polypeptide"** is defined as the product of a comK gene; a transcription factor that acts as the final auto-regulatory control switch prior to competence development; involved with activation of the expression of late competence genes involved in DNA-binding and uptake and in recombination (Liu and Zuber, 1998, Hamoen *et al.,* 1998). Exemplary ComK polypeptides are set forth in SEQ ID NO: 17 and SEQ ID NO: 18.

As used herein, the term **"foreign polynucleotide"** and variations thereof are defined as (A) a polynucleotide that is not native to a *Bacillus licheniformis* cell, (B) a polynucleotide that is native to the *Bacillus licheniformis* cell, but which polynucleotide has been modified through the use of genetic elements which are not natively associated with the polynucleotide *(e.g.,* heterologous promoters, 5' UTRs, 3' UTRs and the like) as isolated from the *Bacillus licheniformis* cell, or (C) the use of native elements that have been manipulated to function in a manner that does not normally occur in the *Bacillus licheniformis* cell.

As used herein, the term **"exogenous DNA"** is defined herein as DNA that is external to a *Bacillus licheniformis* cell.

As used herein, the term **"nucleic acid construct"** refers to a nucleic acid molecule *(e.g.,* a polynucleotide molecule), either single-stranded or double-stranded, which is isolated from a naturally occurring gene or is modified to contain segments of nucleic acids in a manner that would not otherwise exist in nature or is synthetic. The term nucleic acid construct is synonymous with the term "expression cassette" when the nucleic acid construct contains the control sequences required for expression of a coding sequence.

As used herein, the term **"control sequences"** is defined to include all components, which are necessary or advantageous for the expression of a polynucleotide encoding a polypeptide of the present invention. Each control sequence may be native or foreign to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleotide sequence encoding a polypeptide.

As used herein, the term **"promoter"** is defined as a DNA sequence that binds RNA polymerase and directs the polymerase to the correct downstream transcriptional start site of a polynucleotide encoding a polypeptide. RNA polymerase effectively catalyzes the assembly of messenger RNA complementary to the appropriate DNA strand of the coding region. The term "promoter" will also be understood to include the 5' non-coding region (between promoter and translation start) for translation after transcription into mRNA, cis-acting transcription control elements such as enhancers, and/or other nucleotide sequences capable of interacting with transcription factors. The promoter can be a wild-type, variant, hybrid, or a consensus promoter.

As used herein, the term **"promoter region"** is defined as a nucleotide sequence comprising one or more (several) promoter sequences (*e.g*., a dual promoter, a triple promoter and the like).

As used herein, the term **"operably linked"** denotes a configuration in which a control sequence (*e.g*., a promoter sequence) is placed at an appropriate position relative to the coding sequence of the polynucleotide sequence such that the control sequence directs the expression of the coding sequence of a polypeptide. Coding sequence: When used herein the term "coding sequence" means a nucleotide sequence, which directly specifies the amino acid sequence of its protein product. The boundaries of the coding sequence are generally determined by an open reading frame, which usually begins with the ATG start codon or alternative start codons such as GTG and TTG and ends with a stop codon such as TAA, TAG and TGA. The coding sequence may be a DNA, cDNA, synthetic, or recombinant nucleotide sequence.

For purposes of the present disclosure, the degree of identity between two amino acid sequences can be determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et at, 2000, Trends in Genetics 16: 276-277), preferably version 3.0.0 or later. The optional parameters used are gap open penalty of 10, gap extension penalty of 0.5, and the EBLOSUM62 (EMBOSS version of BLOSUM62) substitution matrix.

For purposes of the present disclosure, the degree of identity between two nucleic acid sequences can be determined using the Needleman-Wunsch algorithm (Needleman and Wunsch, 1970, supra) as implemented in the Needle program of the EMBOSS package (EMBOSS: The European Molecular Biology Open Software Suite, Rice et al., 2000, supra), preferably version 3.0.0 or later. The optional parameters used are gap open penalty of 10, gap extension penalty of 0.5, and the EDNAFULL (EMBOSS version of NCBI NUC4.4) substitution matrix.

As used herein, an **"isolated polynucleotide"** refers to a polynucleotide that is isolated from a source. In certain aspects of the disclosure, the polynucleotide is at least 1% pure, preferably at least 5% pure, more preferably at least 10% pure, more preferably at least 20% pure, more preferably at least 40% pure, more preferably at least 60% pure, even more preferably at least 80% pure, and most preferably at least 90% pure, as determined by agarose electrophoresis.

As used herein, a **"substantially pure polynucleotide"** refers to a polynucleotide preparation free of other extraneous or unwanted nucleotides and in a form suitable for use within genetically engineered protein production systems. Thus, a substantially pure polynucleotide contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polynucleotide material with which it is natively or recombinantly associated. A substantially pure polynucleotide may, however, include naturally occurring 5' and 3' untranslated regions, such as promoters and terminators. It is preferred that the substantially pure polynucleotide is at least 90% pure, preferably at least 92% pure, more preferably at least 94% pure, more preferably at least 95% pure, more preferably at least 96% pure, more preferably at least 97% pure, even more preferably at least 98% pure, most preferably at least 99%, and even most preferably at least 99.5% pure by weight. The polynucleotides of the present invention are preferably in a substantially pure form (*i.e*., that the polynucleotide preparation is essentially free of other polynucleotide material with which it is natively or recombinantly associated). The polynucleotides may be of genomic, cDNA, RNA, semisynthetic, synthetic origin, or any combinations thereof.

As used herein, an **"isolated polypeptide"** refers to a polypeptide that is isolated from a source. In a preferred aspect, the polypeptide is at least 1% pure, preferably at least 5% pure, more preferably at least 10% pure, more preferably at least 20% pure, more preferably at least 40% pure, more preferably at least 60% pure, even more preferably at least 80% pure, and most preferably at least 90% pure, as determined by SDS-PAGE.

As used herein, a **"substantially pure polypeptide"** denotes herein a polypeptide preparation that contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polypeptide material with which it is natively or recombinantly associated. It is, therefore, preferred that the substantially pure polypeptide is at least 92% pure, preferably at least 94% pure, more preferably at least 95% pure, more preferably at least 96% pure, more preferably at least 96% pure, more preferably at least 97% pure, more preferably at least 98% pure, even more preferably at least 99%, most preferably at least 99.5% pure, and even most preferably 100% pure by weight of the total polypeptide material present in the preparation. The polypeptides of the present invention are preferably in a substantially pure form (*i*.*e*., that the polypeptide preparation is essentially free of other polypeptide material with which it is natively or recombinantly associated). This can be accomplished, for example, by preparing the polypeptide by well-known recombinant methods or by classical purification methods.

As used herein, a **"coding sequence"** means a nucleotide sequence (*e.g*., a polynucleotide), which directly specifies the amino acid sequence of its protein product. The boundaries of the coding sequence are generally determined by an open reading frame (ORF), which usually begins with the ATG start codon or alternative start codons such as GTG and TTG and ends with a stop codon such as TAA, TAG and TGA. The coding sequence may be a DNA, cDNA, synthetic, or recombinant nucleotide sequence.

As used herein, **"expression"** includes any step involved in the production of a polypeptide of interest (POI) including, but not limited to, transcription, post-transcriptional modification, translation, post-translational modification and secretion.

As used herein, an **"expression vector"** and **"expression construct"** are used interchangeably and refer to a linear or circular DNA molecule that comprises a polynucleotide encoding a polypeptide of interest, and is operably linked to additional nucleotides that provide for its expression.

As used herein, a **"host cell"** includes any cell type that is susceptible to transformation, transfection, transduction, conjugation, and the like with a nucleic acid construct or expression vector.

As used herein, **"transformation"** is defined as introducing an exogenous DNA into a *Bacillus* cell so that the DNA is maintained as a chromosomal integrant or as a self-replicating extra-chromosomal vector.

As used herein, a **"transformant"** generally encompass any *Bacillus* host cell into which an exogenous DNA has been introduced by transformation. The term "transformant" does not include transfectants, conjugants, and transformants generated by an artificial method such as electroporation, protoplasts, heat shock, or CaCl₂ treatment.

### II. ComS Polypeptides and Genes Encoding the Same

As briefly set forth above in the Background Section of the disclosure, many *Bacillus licheniformis* strains and host cells thereof are poorly competent. Recently, certain processes have been developed to improve *B. licheniformis* transformation competency. For example, the overexpression of the transcription factor ComK (*i.e*., expressed from an introduced plasmid containing the *B. licheniformis comK* gene operably linked to an inducible promoter) have been able to partly overcome this poor competence, but transformation efficiencies are still very low (typically less than 20 transformants/transformation). Currently, inefficient *B. licheniformis* DNA transformation (low competency) is still the main bottleneck for the construction of DNA libraries in *B. licheniformis* using direct chromosomal integration. More particularly, the identification of factors that are limiting or inhibiting natural competence is crucial to improve the transformation efficiencies in *B. licheniformis* host cells.

In *Bacilli,* natural competence is a highly complex system of genetic regulation that can be triggered by many external stimuli, such as cell density, stress and quorum sensing (*e.g.*, see Hamoen *et al.,* 2003; Jakobs *et al.,* 2015). The early onset of competence is the triggered by the pheromone "ComX" and the competence stimulating factor ("CSF"). These factors accumulate in the culture medium and induce the membrane bound histidine kinase "ComP" and subsequently its response regulator "ComA". The phosphorylation of the ComA results in a fine-tuned genetic response of the *Bacillus* cell to prepare the cell for competence. Ultimately, the key auto stimulatory competence regulator "ComK" is triggered, which subsequently induces a variety of late competence genes involved in DNA binding and uptake, as well as chromosomal integration (Hamoen *et al.,* 2003; Jakobs *et al.,* 2015). Additionally, the phosphorylated ComA also induces the biotenside surfactin *srf* operon in *Bacillus subtilis* (Roggiani and Dubnau, 1993). More particularly, within the *srf* operon in *B. subtilis* sits a small gene encoding the "ComS" peptide (D'Souza, 1994). This peptide (*i.e*., ComS) is decisive for competence formation since it interacts with "MecA", which recruits ComK towards proteolysis by the ClpCP complex (D'Souza, 1994; Hamoen *et al.,* 1995). In *B. subtilis,* the function of ComS, as well as its induction by the ComP/ComA has been shown to be vital for competence state of the cell (Ashikaga *et al.,* 2000; Nakano and Zuber, 1991).

In contrast to *B. subtilis,* in *Bacillus licheniformis* two putative ComS peptides, named "ComS1" and "ComS2", have been described in the *Ich* (lichenysin synthase) operon (Hoffmann *et al.,* 2010; Jakobs 2015). In addition, the *B. licheniformis* DSM13 type strain can develop a genetic competent state even though the ComP (histidine kinase) is not functional (*i.e*., due to an insertion in the ComP gene), which is opposite (in contrast) to what is observed in *B. subtilis,* (Lapidus *et al.,* 2002). Furthermore, neither the expression of a *B. subtilis* ComS polypeptide in *B. licheniformis,* nor the expression of a *B. licheniformis* ComS1 polypeptide or ComS2 polypeptide in a *comS1* or *comS2* negative *B. licheniformis* background, yielded improved transformation efficiencies (Jakobs, 2015). This result directly conflicts with the surprising and unexpected results described in present disclosure, wherein the expression of the native *comS1* (encoding a comS1 polypeptide of SEQ ID NO: 2) shows a clear competence improvement in *B. licheniformis* host cells (*e.g., see,* Example 1). Furthermore, as presented in Example 1 below, the expression of native *comS2* (encoding a ComS2 polypeptide of SEQ ID NO: 4) in *B. licheniformis* results in reduced competency (*i.e*., in the presence and absence of *comS1*)*.*

Additionally, a comparison of the *B. licheniformis* genomes from a parental *B. licheniformis* (Bra7 strain) host cell and daughter *B. licheniformis* host cells derived therefrom (*i.e., Daughter Cell 1* or *Daughter Cell* 2), revealed that the *B. licheniformis* daughter host cells (*i.e., Daughter Cell 1* and *Daughter Cell* 2) have an approximately 10 kb fragment deleted in the LchAB gene of the *Ich* operon. Without wishing to be bound by a particular theory or mechanism, it is contemplated herein that a recombination event appears to have occurred on the 3'-end of the LchAA and LchAB genes, since there is a region of high homology (*e*.*g*., *see* FIG. 1). More particularly, the genes encoding the ComS1 and ComS2 peptides are located at the 5'-region of the LchAB gene, which in the *B. licheniformis* daughter cells (*i.e., Daughter Cell* 1 and *Daughter Cell 2*) is looped out (*see*, FIG. 2).

Certain methods for obtaining genetic competence in *Bacillus spp.* host cells have been disclosed in PCT Publication No. WO2008/079895. More particularly, the WO2008/079895 reference describes the use of a *comS* gene encoding a ComS polypeptide which is allegedly useful for rendering a non-competent *Bacillus* cells genetically competent. For example, as disclosed in the WO2008/079895 publication, polynucleotides encoding a ComS polypeptide can be obtained from *Bacillus amyloliquefaciens, Bacillus subtilis* or *Bacillus licheniformis,* wherein such encoded ComS polypeptides comprise an amino acid sequence of SEQ ID NO: 2 (*i.e*., ComS polypeptide from *B. subtilis*), SEQ ID NO: 4 (*i.e*., ComS polypeptide from *B. amyloliquefaciens*), SEQ ID NO: 6 (*i.e.*, ComS polypeptide from *B. subtilis natto*), SEQ ID NO: 8 (*i.e*., ComS polypeptide from *B. licheniformis*) or SEQ ID NO: 10 (*i.e*., ComS polypeptide from *B. licheniformis*)*.*

As is presented in FIG. 3A, the ComS1 and ComS2 polypeptides from the parental *B. licheniformis* (Bra7 strain) comprise approximately 50% sequence identity between amino acid residues 36-69 of ComS1 and amino acid residues 21-54 of ComS2. In addition, Applicants of the instant disclosure compared the *Bacillus spp.* amino acid sequences of the ComS polypeptides described in PCT Publication No. WO2008/079895 versus the amino acid sequences of *B. licheniformis* Bra7 strain ComS1 and ComS2 polypeptides of the instant disclosure. As shown in FIG. 3B, the *B. licheniformis* ComS polypeptides disclosed in the WO2008/079895 reference (*i.e*., SEQ ID NO: 8 and SEQ ID NO: 10 in FIG. 1B) correlate to the ComS2 polypeptide from the parental *B. licheniformis* (Bra7 strain) host cells. More particularly, as set forth below in Example 1 of the instant disclosure, the expression of a comS2 polypeptide (*i.e*., comprising SEQ ID NO: 4) in *B. licheniformis* host cells significantly reduced the transformation efficiency of the *B. licheniformis* host cells.

In contrast, the *B. licheniformis* Bra7 strain ComS1 polypeptide of the instant disclosure (*i.e*., comprising SEQ ID NO: 2) shares about 45-50% sequence identity to the *B. subtilis, B. amyloliquefaciens, B. subtilis natto* and *B. licheniformis* ComS polypeptides disclosed in the WO2008/079895 reference (*see*, FIG. 3B). More particularly, as set forth below in Example 1, the expression of only the ComS1 polypeptide (SEQ ID NO: 2) in *B. licheniformis* (daughter) host cells dramatically improved competency by a factor 31.5 at peak competence relative to the same *B. licheniformis* (daughter) host cells which do not comprise (or express) the ComS1 polypeptide.

Thus, in certain embodiments the disclosure is directed to non-competent *Bacillus spp.* host cells which are made competent by the introduction of at least one copy of an exogenous (*i.e*., a foreign polynucleotide) nucleic acid construct comprising a promoter region operably linked to a polynucleotide encoding a ComS1 polypeptide. In certain other embodiments, the disclosure is directed to non-competent *Bacillus licheniformis* host cells which are made competent by the introduction of at least one copy of an exogenous (*i.e*., a foreign polynucleotide) nucleic acid construct comprising a promoter region operably linked to a polynucleotide encoding a ComS1 polypeptide.

In certain embodiments, the ComS1 polypeptide comprises an amino acid sequence comprising at least 90% sequence identity to SEQ ID NO: 2. In other embodiments, a ComS1 polypeptide of SEQ ID NO: 2 is encoded by a *comS1* polynucleotide of SEQ ID NO: 1 or SEQ ID NO: 21.

Certain other embodiments of the disclosure are directed to transforming such B. *licheniformis* host cells made competent by the introduction at least one copy of an exogenous nucleic acid construct comprising a promoter region operably linked to a polynucleotide encoding a ComS1 polypeptide. In certain embodiments, such competent *B. licheniformis* host cells are transformed with a polynucleotide encoding a protein of interest (POI).

In certain other embodiments, the genome of a putative *B. licheniformis* host cell of the disclosure is first analyzed for the presence of a gene encoding a ComS2 polypeptide comprising at least 90% sequence identity to SEQ ID NO: 4. For example, as generally described above and set forth in Example 1, it was observed that the expression of the comS2 polypeptide (*i.e*., comprising SEQ ID NO: 4) in *B. licheniformis* host cells significantly reduced the transformation efficiency of the *B*. *licheniformis* host cells. Thus, in certain embodiments, a putative *B*. *licheniformis* host cell which has been analyzed (and found to comprise a genomic copy of a gene encoding a ComS2 polypeptide comprising at least 90% sequence identity to SEQ ID NO: 4) is genetically modified to reduce ComS2 activity or abolish ComS2 activity. Methods for genetically modifying (*e.g*., deleting, disrupting, interfering, and the like) a *coms2* gene (*e.g*., SEQ ID NO: 3 and SEG ID NO:22) in a *B. licheniformis* host cell are well known to one skilled in the art.

### III. ComK Polypeptides and Genes Encoding the Same

As set forth above, a first nucleic acid construct comprising a promoter region operably linked to a polynucleotide encoding a ComS1 polypeptide comprising 90% sequence identity to SEQ ID NO: 2 is used to render a non-competent *Bacillus licheniformis* host cell genetically competent. In addition, any isolated polynucleotide encoding a ComK polypeptide may be used to render a competent *Bacillus licheniformis* cell genetically more competent *(i.e*., ComK further enhances/increases natural competency). In certain embodiments, a polynucleotide encoding a ComK polypeptide may be introduced into a non-competent *Bacillus licheniformis* host cell before introducing the ComS1 construct or a polynucleotide encoding a comK polypeptide and a polynucleotide encoding comS1 can be introduced into a non-competent *Bacillus licheniformis* host cell at the same time.

Polynucleotides encoding a ComK polypeptide can be obtained from, for example, *Bacillus subtilis* 168 (Accession No. P40396), *Bacillus licheniformis* (DSM 13/ATCC 14580; (Accession No. Q65LN7), *Bacillus licheniformis* (Accession No. Q8VQ66), *Bacillus sp. Bt* 24 (Accession No. Q2HQ42), *Bacillus weihenstephanensis* KBA84 (Accession No. Q2AUN4), *Bacillus thuringiensis* subsp. Konkukian (Accession No. Q6HM51), *Bacillus cereus* (ATCC 10987; (Accession No. Q73C31), *Bacillus cereus* (strain ZK/E33L; (Accession No. Q63EM6), *Bacillus cereus* G9241 (Accession No. Q4MPH9) and the like.

In certain embodiments, a ComK polynucleotide encodes a ComK polypeptide of SEQ ID NO: 17 or SEQ ID NO: 18. The construction of an integrative plasmid comprising a xylR-PxylA-ComK cassette and its transformation into *Bacillus* cells has been described in PCT International Publication No. WO2002/14490.

### IV. Bacillus licheniformis Host Cells

In the compositions and methods of the present disclosure, the *Bacillus licheniformis* host cell may be any non-competent or poorly transformable *Bacillus licheniformis* cell. It is understood that the term *"Bacillus"* herein also encompasses synonyms of *Bacillus* and *genera* formerly classified as *Bacillus* such *Geobacillus* and *Paenibacillus.*

In a further aspect of the present disclosure, the *Bacillus licheniformis* host cells may additionally contain one or more (several) modifications (*e.g*., deletions or disruptions of other genes that may be detrimental to the production, recovery, or application of a polypeptide or biochemical of interest. Thus, in certain embodiments, the *Bacillus licheniformis* host cell is a protease-deficient cell, an amylase-deficient cell, *etc.* For example, in certain embodiments, the *Bacillus licheniformis* host cell comprises a disruption or deletion of *aprE, nprE, amyE, amyL,* and the like. In another embodiment, the *Bacillus* host cell does not produce spores (*e.g*., comprising a disruption or deletion of spollAC). In other embodiments, the *Bacillus licheniformis* host cell comprises a disruption or deletion of one or more genes involved in the biosynthesis of surfactin/lichenysin *(e.g., srfA, srfB, srfC, srfD, lchAA, lchAB, lchAC and the like)* or complete deletion of the *srf* operon or *Ich* operon. In other embodiments, particularly when complementing the *Bacillus licheniformis* host cell with a ComS1 polypeptide (SEQ ID NO: 2) of the disclosure, the *srf* operon or the *Ich* operon is deleted in the host cell.

Thus, certain embodiments of the disclosure relate to competent *Bacillus licheniformis* host cells comprising at least one copy of an introduced nucleic acid construct comprising a promoter region operably linked to a polynucleotide encoding a ComS1 polypeptide comprising 90% sequence identity to SEQ ID NO: 2. wherein the *Bacillus licheniformis* host cell was non-competent prior to introduction of the ComS1 nucleic acid construct.

For example, as contemplated herein, in certain embodiments, a *Bacillus licheniformis* host cell which does not natively comprise a gene encoding a ComS1 polypeptide comprising 90% sequence identity to SEQ ID NO: 2, is modified by introducing at least one copy a nucleic acid construct comprising a promoter region operably linked to a polynucleotide encoding a ComS1 polypeptide comprising 90% sequence identity to SEQ ID NO: 2. More particularly, as described herein, the introduction of the above ComS1 construct into a *Bacillus* spp. host cell lacking a native gene encoding a ComS1 polypeptide enhances natural competence of the *Bacillus licheniformis* host cell.

Likewise, in certain other embodiments, a *Bacillus licheniformis* host cell comprising a native gene encoding a ComS1 polypeptide *(e.g., Bacillus licheniformis* Bra7 strain) or comprising a truncated or deleted ComS1 gene *(e.g*., daughter cells of *Bacillus licheniformis* Bra7 strain) is modified by introducing at least one copy a nucleic acid construct comprising a promoter region operably linked to a polynucleotide encoding a ComS1 polypeptide comprising 90% sequence identity to SEQ ID NO: 2. For example, as presented in Example 1 below, the introduction of a ComS1 construct into a non-competent (or poorly transformable) *B. licheniformis* host cell lacking a native gene encoding a ComS1 polypeptide *(e.g., B. licheniformis* daughter cells derived from parental *B. licheniformis* Bra7 strain) renders such non-competent host cells competent.

Thus, as contemplated and disclosed herein, a nucleic acid construct of the present disclosure, comprising a promoter region operably linked to a polynucleotide encoding a ComS1 polypeptide comprising 90% sequence identity to SEQ ID NO: 2, finds particular utility for rending non-competent *Bacillus licheniformis* host cells competent. Likewise, a nucleic acid construct of the present disclosure, comprising a promoter region operably linked to a polynucleotide encoding a ComS1 polypeptide comprising 90% sequence identity to SEQ ID NO: 2 finds utility in enhancing/increasing competence in *Bacillus licheniformis* host cells which comprise an endogenous gene encoding a ComS1 polypeptide. In certain embodiments, the *Bacillus licheniformis* host cells made competent by introducing the nucleic acid construct comprising a promoter region operably linked to a polynucleotide encoding a ComS1 polypeptide comprising 90% sequence identity to SEQ ID NO: 2 increases the number of Bacilli transformants by at least 1.5-fold, or at least 2-fold, or at least 5-fold, or at least 10-fold, or at least 50-fold or higher.

In other embodiments, a *Bacillus licheniformis* host cell made competent as described above, further comprises at least one copy of an introduced second nucleic acid construct comprising a promoter region operably linked to a polynucleotide encoding a ComK polypeptide to render the *Bacillus licheniformis* host cell even further competent above the competence obtained by expression of a ComS1 polypeptide.

In certain other embodiments, a *B. licheniformis* host cell of the disclosure comprises a genetic modification which reduces ComS2 activity or abolishes ComS2 activity, which genetic modification methods are well known to one skilled in the art.

In particular embodiments, the competent *Bacillus licheniformis* host cells further comprise a nucleic acid construct or recombinant expression vector comprising a DNA or gene of interest encoding a protein of interest.

### V. Expression of ComS1 and ComK Polynucleotides in Bacillus licheniformis Host Cells

A polynucleotide encoding a ComS1 polypeptide or a ComK polypeptide can be manipulated in a variety of ways to provide for expression of the polynucleotide in a *Bacillus licheniformis* host cell. Manipulation of the polynucleotide sequence prior to its insertion into a nucleic acid construct or vector may be desirable or necessary depending on the nucleic acid construct or the *Bacillus licheniformis* host cell. The techniques for modifying nucleotide sequences utilizing cloning methods are well known in the art.

A nucleic acid construct comprising a polynucleotide encoding a ComS1 polypeptide or a ComK polypeptide may be operably linked to one or more control sequences capable of directing the expression of the coding sequence in a *Bacillus* host cell under conditions compatible with the control sequences.

Each control sequence may be native or foreign to the polynucleotide encoding a ComS1 polypeptide or a ComK polypeptide. Such control sequences include, but are not limited to, a leader sequence, a promoter sequence, a signal sequence, and a transcription terminator sequence. At a minimum, the control sequences include a promoter and, transcriptional and translational stop signals. The control sequences may be provided with linkers for the purpose of introducing specific restriction sites facilitating ligation of the control sequences with the coding region of the polynucleotide encoding the ComS1 polypeptide or the ComK polypeptide.

The control sequence may be an appropriate promoter region, a nucleotide sequence that is recognized by a *Bacillus licheniformis* host cell for expression of the polynucleotide encoding a ComS1 polypeptide or a ComK polypeptide. The promoter region contains transcription control sequences that mediate the expression of a ComS1 polypeptide or a ComK polypeptide. The promoter region may be any nucleotide sequence that shows transcriptional activity in the *Bacillus licheniformis* host cell of choice and may be obtained from genes directing synthesis of extracellular or intracellular polypeptides having biological activity either homologous or heterologous to the *Bacillus licheniformis* host cell.

The promoter region may comprise a single promoter or a combination of promoters. Where the promoter region comprises a combination of promoters, the promoters are preferably in tandem. A promoter of the promoter region can be any promoter that can initiate transcription of a polynucleotide encoding a polypeptide having biological activity in a *Bacillus licheniformis* host cell of interest. The promoter may be native, foreign, or a combination thereof, to the nucleotide sequence encoding a polypeptide having biological activity. Such a promoter can be obtained from genes directing synthesis of extracellular or intracellular polypeptides having biological activity either homologous or heterologous to the *Bacillus licheniformis* host cell.

Thus, in certain embodiments, the promoter region comprises a promoter obtained from a bacterial source. In other embodiments, the promoter region comprises a promoter obtained from a Gram positive or Gram negative bacterium. Gram positive bacteria include, but are not limited to, *Bacillus, Streptococcus, Streptomyces, Staphylococcus, Enterococcus, Lactobacillus, Lactococcus, Clostridium, Geobacillus,* and *Oceanobacillus.* Gram negative bacteria include, but are not limited to, *E. coli, Pseudomonas, Salmonella, Campylobacter, Helicobacter, Flavobacterium, Fusobacterium, Ilyobacter, Neisseria,* and *Ureaplasma.*

In certain embodiments, the promoter region comprises a promoter obtained from a *Bacillus* species or strain thereof *(e.g., Bacillus agaradherens, Bacillus alkalophilus, Bacillus amyloliquefaciens, Bacillus brevis, Bacillus circulans, Bacillus clausii, Bacillus coagulans, Bacillus firm us, Bacillus lautus, Bacillus lentus, Bacillus licheniformis, Bacillus megaterium, Bacillus pumilus, Bacillus stearothermophilus, Bacillus subtilis,* or *Bacillus thuringiensis;* or from a *Streptomyces* strain *(e.g., Streptomyces lividans* or *Streptomyces murinus).*

Examples of suitable promoters for directing transcription of a polynucleotide encoding a polypeptide having biological activity in the methods of the present disclosure are the promoters obtained from the *E. coli lac* operon, *Streptomyces* coelicolor agarase gene *(dagA), Bacillus lentus* or *Bacillus clausii* alkaline protease gene *(aprH), Bacillus licheniformis* alkaline protease gene (subtilisin Carlsberg gene), *Bacillus subtilis* levansucrase gene *(sacB), Bacillus subtilis* alpha-amylase gene *(amyE), Bacillus licheniformis* alpha-amylase gene *(amyL), Bacillus stearothermophilus* maltogenic amylase gene *(amyM), Bacillus amyloliquefaciens* alpha-amylase gene (amyQ), *Bacillus licheniformis* penicillinase gene *(penP), Bacillus subtilis xylA* and *xylB* genes, *Bacillus thuringiensis* subsp. tenebfionis CrylllA gene *(cryIIIA)* or portions thereof, prokaryotic beta-lactamase gene (Villa-Kamaroff *et al.,* 1978), and *Bacillus megaterium* xylA gene (Rygus and Hillen, 1992; Kim *et al.,* 1996). Other examples are the promoter of the spot bacterial phage promoter and the tac promoter (DeBoer *et al.,* 1983). Further promoters are described in Sambrook, Fritsch, and Maniatus (1989).

In certain embodiments, a nucleic acid construct of the present disclosure, comprising a promoter region operably linked to a polynucleotide encoding a ComS1 polypeptide comprising 90% sequence identity to SEQ ID NO: 2. In certain embodiments, a polynucleotide encoding a ComS1 polypeptide comprises a promoter region set forth in SEQ ID NO: 5. In certain other embodiments, a polynucleotide encoding a ComS1 polypeptide comprises a nucleic acid terminator sequence of SEQ ID NO: 6.

In another embodiment, the promoter region comprises a promoter that is a "consensus" promoter having the sequence TTGACA for the "-35" region and TATAAT for the "-10" region. The consensus promoter may be obtained from any promoter that can function in a *Bacillus licheniformis* host cell. The construction of a "consensus" promoter may be accomplished by site-directed mutagenesis using methods well known in the art to create a promoter that conforms more perfectly to the established consensus sequences for the "-10" and "-35" regions of the vegetative "sigma A-type" promoters for *Bacillus subtilis* (Voskuil *et* al., 1995).

In certain other embodiments, a control sequence may also be a suitable transcription terminator sequence, such as a sequence recognized by a *Bacillus licheniformis* host cell to terminate transcription (*e.g*., SEQ ID NO; 6). The terminator sequence is operably linked to the 3' terminus of the nucleotide sequence encoding a ComS1 polypeptide or a ComK polypeptide. Any terminator that is functional in the *Bacillus licheniformis* host cell may be used in the present invention.

The control sequence may also be a suitable leader sequence, a non-translated region of a mRNA that is important for translation by a *Bacillus licheniformis* host cell. The leader sequence is operably linked to the 5' terminus of the nucleotide sequence directing synthesis of the polypeptide having biological activity. Any leader sequence that is functional in a *Bacillus licheniformis* host cell of choice may be used in the present invention.

The control sequence may also be a mRNA stabilizing sequence. The term "mRNA stabilizing sequence" is defined herein as a sequence located downstream of a promoter region and upstream of a coding sequence of a polynucleotide encoding a ComS1 polypeptide or ComK polypeptide to which the promoter region is operably linked, such that all mRNAs synthesized from the promoter region may be processed to generate mRNA transcripts with a stabilizer sequence at the 5' end of the transcripts. For example, the presence of such a stabilizer sequence at the 5' end of the mRNA transcripts increases their half-life (Hue *et al.,* 1995). The mRNA processing/stabilizing sequence is complementary to the 3' extremity of bacterial 16S ribosomal RNA. In certain embodiments, the mRNA processing/stabilizing sequence generates essentially single-size transcripts with a stabilizing sequence at the 5' end of the transcripts. The mRNA processing/stabilizing sequence is preferably one, which is complementary to the 3' extremity of a bacterial 16S ribosomal RNA *(e.g., see,* U.S. Patent No. 6,255,076 and U.S. Patent No. 5,955,310).

The nucleic acid construct can then be introduced into a *Bacillus licheniformis* host cell using methods known in the art or those methods described herein for introducing and expressing the ComS1 polypeptide or ComK polypeptide.

Likewise, a nucleic acid (polynucleotide) construct comprising a DNA of interest encoding a protein of interest (POI) can also be constructed similarly as described above.

For obtaining secretion of the protein of interest of encoded by the introduced DNA of interest, the control sequence may also comprise a signal peptide coding region, which codes for an amino acid sequence linked to the amino terminus of a polypeptide that can direct the expressed polypeptide into the cell's secretory pathway. The signal peptide coding region may be native to the polypeptide or may be obtained from foreign sources. The 5' end of the coding sequence of the nucleotide sequence may inherently contain a signal peptide coding region naturally linked in translation reading frame with the segment of the coding region that encodes the secreted polypeptide. Alternatively, the 5' end of the coding sequence may contain a signal peptide coding region that is foreign to that portion of the coding sequence that encodes the secreted polypeptide. The foreign signal peptide coding region may be required where the coding sequence does not normally contain a signal peptide coding region. Alternatively, the foreign signal peptide coding region may simply replace the natural signal peptide coding region in order to obtain enhanced secretion of the polypeptide relative to the natural signal peptide coding region normally associated with the coding sequence. The signal peptide coding region may be obtained from an amylase or a protease gene from a *Bacillus* species. However, any signal peptide coding region capable of directing the expressed polypeptide into the secretory pathway of a *Bacillus licheniformis* host cell of choice may be used in the present invention.

An effective signal peptide coding region for a *Bacillus licheniformis* host cell, is the signal peptide coding region obtained from the maltogenic amylase gene from *Bacillus* NCIB 11837, the *Bacillus stearothermophilus* alpha-amylase gene, the *Bacillus licheniformis* subtilisin gene, *Bacillus licheniformis amyL* gene, the *Bacillus licheniformis* β-lactamase gene, the *Bacillus stearothermophilus* neutral proteases genes *(nprT, nprS, nprM),* and the *Bacillus subtilis prsA* gene.

Thus, in certain embodiments, a polynucleotide construct comprising a nucleic acid encoding a ComS1 polypeptide, or a polynucleotide construct comprising a nucleic acid encoding a ComK polypeptide or a polynucleotide construct comprising a nucleic acid encoding a polypeptide of interest (POI) of the disclosure, can be constructed such that it is expressed by a *Bacillus* host cell. Because of the known degeneracies in the genetic code, different polynucleotides encoding an identical amino acid sequence can be designed and made with routine skills in the art. For example, codon optimizations can be applied to optimize production in a *Bacillus* host cell.

Nucleic acids encoding a ComS1 polypeptide, a ComK polypeptide and/or a protein of interest *(see*, Section VI below) can be incorporated into a vector, wherein the vector can be transferred into a host cell using well-known transformation techniques, such as those disclosed herein.

The vector may be any vector that can be transformed into and replicated within a host cell. For example, a vector comprising a nucleic acid encoding a POI can be transformed and replicated in a bacterial host cell as a means of propagating and amplifying the vector. The vector also may be transformed into a *Bacillus licheniformis* expression host of the disclosure, so that the protein encoding nucleic acid *(e.g*., an ORF) can be expressed as a functional protein.

A representative vector which can be modified with routine skill to comprise and express a nucleic acid encoding a POI is vector p2JM103BBI *(see*, Vogtentanz, 2007).

As stated briefly above, a polynucleotide encoding a ComS1 polypeptide, a comK polypeptide or a POI can be operably linked to a suitable promoter, which allows transcription in the host cell. The promoter may be any nucleic acid sequence that shows transcriptional activity in the host cell of choice and may be derived from genes encoding proteins either homologous or heterologous to the host cell. Means of assessing promoter activity/strength are routine for the skilled artisan.

Examples of suitable promoters for directing the transcription of a polynucleotide sequence encoding ComS1 polypeptide, a ComK polypeptide and/or a POI of the disclosure, especially in a bacterial host, include the promoter of the lac operon of *E. coli,* the *Streptomyces coelicolor* agarase gene *dagA* or *celA* promoters, the promoters of the *Bacillus licheniformis* alpha-amylase gene *(amyL),* the promoters of the *Bacillus stearothermophilus* maltogenic amylase gene *(amyM),* the promoters of the *Bacillus amyloliquefaciens* alpha-amylase *(amyQ),* the promoters of the *Bacillus subtilis xylA* and *xylB* genes, and the like.

In certain embodiments, a promoter for directing the transcription of a polynucleotide sequence encoding a POI is a wild-type *aprE* promoter, a mutant *aprE* promoter or a consensus *aprE* promoter set forth in PCT International Publication No. WO2001/51643. In certain other embodiments, a promoter for directing the transcription of a polynucleotide sequence encoding a POI is a wild-type *spoVG* promoter, a mutant *spoVG* promoter, or a consensus *spoVG* promoter (Frisby and Zuber, 1991).

In other embodiments, a promoter for directing the transcription of the polynucleotide sequence encoding ComS1 polypeptide, a ComK polypeptide or a POI is a ribosomal promoter such as a ribosomal RNA promoter or a ribosomal protein promoter. More particularly, in certain embodiments, the ribosomal RNA promoter is a *rrn* promoter derived from *B. subtilis,* more particularly, the *rrn* promoter is a *rrnB, rrnI* or *rrnE* ribosomal promoter from *B. subtilis.* In certain embodiments, the ribosomal RNA promoter is a P2 *rrnI* promoter from *B. subtilis* set forth in PCT International Publication No. WO2013/086219.

A suitable vector may further comprise a nucleic acid sequence enabling the vector to replicate in the host cell. Examples of such enabling sequences include the origins of replication of plasmids pUC19, pACYC177, pUB110, pE194, pAMB1, pIJ702, and the like.

A suitable vector may also comprise a selectable marker, *e.g.,* a gene the product of which complements a defect in the isolated host cell, such as the *daI* genes from *B. subtilis* or *B. licheniformis;* or a gene that confers antibiotic resistance such as, *e.g.,* ampicillin resistance, kanamycin resistance, chloramphenicol resistance, tetracycline resistance and the like.

A suitable expression vector typically includes components of a cloning vector, such as, for example, an element that permits autonomous replication of the vector in the selected host organism and one or more phenotypically detectable markers for selection purposes. Expression vectors typically also comprise control nucleotide sequences such as, for example, promoter, operator, ribosome binding site, translation initiation signal and optionally, a repressor gene, one or more activator genes sequences, or the like.

Additionally, a suitable expression vector may further comprise a sequence coding for an amino acid sequence capable of targeting the protein of interest to a host cell organelle such as a peroxisome, or to a particular host cell compartment. Such a targeting sequence may be, for example, the amino acid sequence "SKL". For expression under the direction of control sequences, the nucleic acid sequence of the protein of interest can be operably linked to the control sequences in a suitable manner such that the expression takes place.

Protocols, such as described herein, used to ligate the DNA construct encoding a protein of interest, promoters, terminators and/or other elements, and to insert them into suitable vectors containing the information necessary for replication, are well known to persons skilled in the art *(see, e.g.,* Sambrook *et al.,* 1989, and 3rd edition 2001).

An isolated cell, either comprising a polynucleotide construct or an expression vector, is advantageously used as a host cell in the recombinant production of a POI. The cell may be transformed with the DNA construct encoding the POI, conveniently by integrating the construct (in one or more copies) into the host chromosome. Integration is generally deemed an advantage, as the DNA sequence thus introduced is more likely to be stably maintained in the cell. Integration of the DNA constructs into the host chromosome may be performed applying conventional methods, for example, by homologous or heterologous recombination. For example, PCT International Publication No. WO2002/14490 describes methods of *Bacillus* transformation, transformants thereof and libraries thereof. Alternatively, the cell may be transformed with an expression vector as described above in connection with the different types of host cells.

It is, in other embodiments, advantageous to delete genes from expression hosts, where the gene deficiency can be cured by an expression vector. Known methods may be used to obtain a bacterial host cell having one or more inactivated genes. Gene inactivation may be accomplished by complete or partial deletion, by insertional inactivation or by any other means that renders a gene nonfunctional for its intended purpose, such that the gene is prevented from expression of a functional protein. For example, in certain embodiments, a *Bacillus licheniformis* host cell of the disclosure comprises either a disrupted or deleted *comS2* gene encoding a ComS2 polypeptide comprising at least 90% sequence identity to the *Bacillus licheniformis* ComS2 polypeptide of SEQ ID NO: 4. Thus, in certain embodiments, a non-competent (or poorly transformable) *Bacillus licheniformis* host cell is made competent by a reduction in the activity of a ComS2 polypeptide in the host cell. In other embodiments, a *Bacillus licheniformis* host cell comprising a deleted or disrupted *comS2* gene further comprises an introduced nucleic acid construct comprising a polynucleotide construct encoding a ComS1 polypeptide comprising 90% sequence identity to SEQ ID NO: 2 and/or an introduced nucleic acid construct comprising a polynucleotide construct encoding a ComK polypeptide (e.g., SEQ ID NO: 17 or SEQ ID NO: 18).

Techniques for transformation of bacteria and culturing the bacteria are standard and well known in the art. They can be used to transform the improved hosts of the present invention for the production of a recombinant protein of interest (POI). Introduction of a DNA construct or vector into a host cell includes techniques such as transformation, electroporation, nuclear microinjection, transduction, transfection (e.g., lipofection mediated and DEAE-Dextrin mediated transfection), incubation with calcium phosphate DNA precipitate, high velocity bombardment with DNA-coated microprojectiles, gene gun or biolistic transformation and protoplast fusion, and the like. Transformation and expression methods for bacteria are also disclosed in Brigidi *et al.* (1990). A general transformation and expression protocol for protease deleted *Bacillus* strains is described in Ferrari et al. (U.S. Patent No. 5, 264,366).

### VI. Proteins of Interest (POI) Produced by Bacillus licheniformis Cells

In certain embodiments, the present disclosure provides methods for producing one or more proteins of interest in one or more *Bacillus licheniformis* host cells made competent according to the instant disclosure. Thus, certain embodiments are directed to *Bacillus licheniformis* host cells made competent *via* introducing a nucleic acid construct encoding a ComS1 polypeptide therein, wherein the competent *Bacillus licheniformis* host cells are transformed with a nucleic acid construct encoding a protein of interest.

The protein of interest (POI) can be any endogenous or heterologous protein, and it may be a variant of such a POI. The protein can contain one or more disulfide bridges or is a protein whose functional form is a monomer or a multimer, i.e., the protein has a quaternary structure and is composed of a plurality of identical (homologous) or non-identical (heterologous) subunits, wherein the POI or a variant POI thereof is preferably one with properties of interest.

In certain embodiments, a POI or a variant POI thereof is selected from the group consisting of acetyl esterases, aryl esterases, aminopeptidases, amylases, arabinases, arabinofuranosidases, carbonic anhydrases, carboxypeptidases, catalases, cellulases, chitinases, chymosins, cutinases, deoxyribonucleases, epimerases, esterases, α-galactosidases, β-galactosidases, α-glucanases, glucan lysases, endo-β-glucanases, glucoamylases, glucose oxidases, α-glucosidases, β-glucosidases, glucuronidases, glycosyl hydrolases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, phenol oxidases, peroxidases, phenoloxidases, phytases, polygalacturonases, proteases, peptidases, rhamno-galacturonases, ribonucleases, transferases, transport proteins, transglutaminases, xylanases, hexose oxidases, and combinations thereof.

The POI or variant POI may also be a peptide, a peptide hormone, a growth factor, a clotting factor, a chemokine, a cytokine, a lymphokine, an antibody, a receptor, an adhesion molecule, a microbial antigen *(e.g*., HBV surface antigen, HPV E7, *etc.)*, and variants thereof or fragments thereof.

### VII. FERMENTATION

In certain embodiments, the disclosure is directed to compositions and methods of producing a POI comprising fermenting a *Bacillus licheniformis* host cell made competent according to the instant disclosure, wherein the competent *Bacillus licheniformis* host cell secrets the POI into the culture medium. Fermentation methods well known in the art can be applied to ferment the *Bacillus licheniformis* host cells of the disclosure.

In some embodiments, the *Bacillus licheniformis* host cells are cultured under batch or continuous fermentation conditions. A classical batch fermentation is a closed system, where the composition of the medium is set at the beginning of the fermentation and is not altered during the fermentation. At the beginning of the fermentation, the medium is inoculated with the desired organism(s). In this method, fermentation is permitted to occur without the addition of any components to the system. Typically, a batch fermentation qualifies as a "batch" with respect to the addition of the carbon source, and attempts are often made to control factors such as pH and oxygen concentration. The metabolite and biomass compositions of the batch system change constantly up to the time the fermentation is stopped. Within typical batch cultures, cells can progress through a static lag phase to a high growth log phase, and finally to a stationary phase, where growth rate is diminished or halted. If untreated, cells in the stationary phase eventually die. In general, cells in log phase are responsible for the bulk of production of product.

A suitable variation on the standard batch system is the "fed-batch fermentation" system. In this variation of a typical batch system, the substrate is added in increments as the fermentation progresses. Fed-batch systems are useful when catabolite repression likely inhibits the metabolism of the cells and where it is desirable to have limited amounts of substrate in the medium. Measurement of the actual substrate concentration in fed-batch systems is difficult and is therefore estimated on the basis of the changes of measurable factors, such as pH, dissolved oxygen and the partial pressure of waste gases, such as CO₂. Batch and fed-batch fermentations are common and known in the art.

Continuous fermentation is an open system where a defined fermentation medium is added continuously to a bioreactor, and an equal amount of conditioned medium is removed simultaneously for processing. Continuous fermentation generally maintains the cultures at a constant high density, where cells are primarily in log phase growth. Continuous fermentation allows for the modulation of one or more factors that affect cell growth and/or product concentration. For example, in one embodiment, a limiting nutrient, such as the carbon source or nitrogen source, is maintained at a fixed rate and all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to medium being drawn off should be balanced against the cell growth rate in the fermentation. Methods of modulating nutrients and growth factors for continuous fermentation processes, as well as techniques for maximizing the rate of product formation, are well known in the art of industrial microbiology.

Thus, in certain embodiments, a POI produced by a transformed *Bacillus licheniformis* cell may be recovered from the culture medium by conventional procedures including separating the host cells from the medium by centrifugation or filtration, or if necessary, disrupting the cells and removing the supernatant from the cellular fraction and debris. Typically, after clarification, the proteinaceous components of the supernatant or filtrate are precipitated by means of a salt *(e.g*., ammonium sulfate). The precipitated proteins are then solubilized and may be purified by a variety of chromatographic procedures *(e.g.,* ion exchange chromatography, gel filtration).

### EXAMPLES

Certain aspects of the present invention may be further understood in light of the following examples, which should not be construed as limiting. Modifications to materials and methods will be apparent to those skilled in the art.

### EXAMPLE 1

### NON-COMPETENT B. LICHENIFORMIS HOST CELLS MADE COMPETENT BY INTRODUCING AN EXPRESSION CONSTRUCT ENCODING NATIVE COMS1 PEPTIDE

The present example evaluates the influence/role that ComS peptides have on the development of natural competence in *B. licheniformis* host cells.

### Materials and Methods

### Plasmid construction

To evaluate the role that the ComS1 (SEQ ID NO: 2) and ComS2 (SEQ ID NO: 4) peptides from *B. licheniformis* (Bra7 strain) play in the development of natural competence in *Bacillus licheniformis* host cells, the following three constructs were prepared: **(1) Construct #1:** a *comS1* construct driven by a promotor and 5' UTR of SEQ ID NO: 5 and a terminator of SEQ ID NO: 6, **(2) Construct #2:** a *comS2* construct driven by a promotor and 5' UTR of SEQ ID NO: 5 and a terminator of SEQ ID NO: 6 and **(3) Construct #3:** a *comS1comS2* construct driven by a promotor and 5' UTR of SEQ ID NO: 5, a terminator of SEQ ID NO: 6, with an additional ribosomal binding (RBS) site of SEQ ID NO: 7 operably linking the *comS1* and *comS2* genes.

All three constructs set forth above were flanked upstream and downstream by a 5' flanking sequence (SEQ ID NO: 19) and a 3' flanking sequence (SEQ ID NO: 20), which are homologous regions of the *amyL* locus of the parental *B. licheniformis* host cell, and ordered synthetically flanked by a *"XhoI"* and a *"NotI"* restriction site (IDT, Leuven, Belgium).

The three (3) synthetic constructs (i.e., Construct #1, Construct #2 and Construct #3) were digested using *'XhoI"* and *"NotI"* (BIOKE, Leiden The Netherland), for two (2) hours at 37°C, and subsequently gel purified (BIOKE, Leiden The Netherland). The isolated digested fragment was ligated into a *"XhoI"* and *"NotI"* digested and gel purified plasmid vector "pCZ1 05" *(see,* FIG. 3). The ligation was performed using the quick ligation^{™} kit (BIOKE, Leiden The Netherland), and the ligate was purified using the PCR cleanup kit (BIOKE, Leiden The Netherland) and rolling circle amplified (GE Healthcare Europe GmbH, Eindhoven, the Netherlands).

The rolling circle amplified ("RCA") mix of the pCZ105 plasmid harboring *comS1* Construct #1) and the pCZ105 plasmid harboring *comS1comS2 (i.e.,* Construct #3) were transformed into competent *B. licheniformis* (daughter) host cells *(i.e., "Daughter Cell 1"),* which daughter host cells do not comprise endogenous functional genes encoding either ComS1 or ComS2. Similarly, the pCZ105 plasmid harboring *comS2 (i.e.,* Construct #2) was transformed into competent *B. licheniformis* (daughter) host cells *(i.e., "Daughter Cell 2"),* which daughter host cells do not comprise functional genes encoding either ComS1 or ComS2. More particularly, the pCZ105 plasmids harboring Construct #1, Construct #2 and Construct #3, were transformed into competent *B. licheniformis* (daughter) cells comprising (harboring) a pBLcomK plasmid (see, FIG. 5) as generally described in International PCT Application No. PCT/US2016/059078 (filed October 27, 2016), and International PCT Publication Nos. WO2002/14490 and WO2008/79895.

All transformations were plated on Luria agar containing 30 mg/l kanamycin and incubated at 37°C. Formed colonies were inoculated in Luria broth containing 30 mg/l kanamycin and incubated overnight at 42°C to promote single crossover integration in the genome, followed by plating on Luria agar containing 30 mg/l kanamycin. Single colonies were checked for correct integration in the genome using the following forward primer and one of the two reverse primers set forth below:
Forward primer: AAGCTGCTGTCCCTTCAAGA (SEQ ID NO: 8);
Reverse primer: CTTCGAGCTGATTGAGGATGTAC (SEQ ID NO: 9); was used for *comS1* and *comS1comS2; and*
Reverse primer: CATGAGAACAGACGGCATGTT (SEQ ID NO: 10); was used for *comS2.*

Double crossover integration was obtained by culturing the correct single crossover integrants in Luria broth overnight at 37°C followed by plating onto Luria agar. Colonies unable to grow in the presence of 30 mg/L Kanamycin were tested by PCR for double crossover integration using primers:
Forward primer: AAGCTGCTGTCCCTTCAAGA (SEQ ID NO: 11)
Reversed primer: CGGCGGTAACAGGAACAATC (SEQ ID NO: 12)

After verification, three (3) different modified *B. licheniformis* host cells were obtained from the daughter *B. licheniformis* host cells transformed as described above: (1) *"Daughter Cell 1"* comprising Construct #1 *(comS1);* (2) *"Daughter Cell 1"* comprising Construct #3 *(comS1comS2)* and (3) *"Daughter Cell 2"* comprising Construct # 2 *(comS2).* These three (3) modified (daughter) cells were cultured to obtain protoplasts and re-transformed with the pBLComK plasmid *(see,* PCT International Publication No. WO2005/111203 and PCT International Application No. PCT/US2016/059078). The DNA and protoplasts mixture were plated on a *B. Licheniformis* regeneration media plate with 100 ppm of spectinomycin as selection. After (3) three days of growth on the regeneration plate, a sample from an individual transformant colony was picked and re-streaked on LB plate with 100 ppm of spectinomycin. Verification of the presence of the pBLComK plasmid was performed using the following primers:
Forward primer 1: TCTCCAAGATAACTACGAACTGC (SEQ ID NO: 13)
Reverse primer 1: CCATAAACCGCCGATCATGG (SEQ ID NO: 14)
Forward primer 2: CGTACGGAGATTGGGGCAT (SEQ ID NO: 15)
Reverse primer 2: ACTCGTGAACATGCGCAT (SEQ ID NO: 16)

### Transformation Efficiency Assay

In the present example, the transformation efficiency of the modified *B. licheniformis* (daughter) host cells described above *(i.e.,* comprising Construct #1, Construct #2 or Construct #3) were analyzed for their ability to take up a replicating plasmid comprising a gene of interest encoding a *G. stearothermophilus* α-amylase variant GC358 (as described in U.S. Patent No. 8,361,755 and U.S. Patent No. 5,958,739).

Thus, from a -80°C stock, the following host cells were evaluated: **(A)** a *B*. *licheniformis* host cell *(Daughter Cell 1)* comprising plasmid pBLComK, **(B)** a *B. licheniformis* host cell *(Daughter Cell 2)* comprising plasmid pBLComK, **(C)** a *B. licheniformis* host cell *(Daughter Cell 1)* comprising *comS1* (Construct #1) and plasmid pBLComK, **(D)** a *B. licheniformis* host cell *(Daughter Cell 1)* comprising *comS1comS2* (Construct #3) and plasmid pBLComK and **(E)** a *B. licheniformis* host cell *(Daughter Cell 2)* comprising *comS2 (Construct #2)* and plasmid pBLComK.

The *B. licheniformis* host cells (A)-(E) described above were plated onto LB plates containing 100mg/L spectinomycin and cultured overnight at 37°C for analysis of competence. More particularly, competence of *B. licheniformis* host cells (A)-(E) was assayed (as described in PCT International Application No. PCT/US2016/059078) at the 3, 4, 5, 6, 7 and 8-hour time points.

### Results

As depicted in FIG. 6A, the expression of the ComS2 peptide (Construct #2) in the *B. licheniformis* host cell *(Daughter Cell 2)* significantly reduced the transformation efficiency of *Daughter Cell 2* relative to the same *B. licheniformis* host cell *(Daughter Cell 2)* which does not comprise Construct #2 *(see,* FIG. 6A, right y-axis). As presented in FIG. 6B, the expression of the combination of the ComS1 and ComS2 peptides (Construct #3) improved competency of *B. licheniformis* host cell *(Daughter Cell 1)* by a factor of 11 at peak competence, relative to the same *B. licheniformis* host cell *(Daughter Cell 1)* which does not comprise Construct #3 *(see,* FIG. 6B, right y-axis). Most surprisingly, as presented in FIG. 6C, the expression of only the ComS1 peptide (Construct #1) in the *B. licheniformis* host cell *(Daughter Cell 1)* dramatically improved competency by a factor 31.5 at peak competence, relative to the same *B. licheniformis* host cell *(Daughter Cell* 1) which does not comprise Construct #1 (ComS1).

Thus, the results presented herein show that the ComS2 peptide (SEQ ID NO: 4), which was annotated based on homology with the *comS* gene from *B. subtilis,* has a negative impact on competency in *B. licheniformis* host cells. In contrast, the expression of only the *comS1* gene in *B. licheniformis* daughter cells significantly enhances *B. licheniformis* competency.

More particularly, the increased competency observed for the expression of the ComS1 peptide (SEQ ID NO: 2) in *B. licheniformis* host cells *(Daughter Cell 1)* is even slightly higher at time point 7, relative to the competency observed for the parental *B. licheniformis* (Bra7 strain) host cell (FIG. 7, right y-axis "Bra7 parent"), which wild-type Bra7 strain natively comprises both genes encoding the ComS1 and ComS2 peptides.

### REFERENCES

PCT International Application No. PCT/US2016/059078
PCT International Application No. PCT/US2016/059078
PCT International Publication No WO2005/111203
PCT International Publication No. WO2001/51643
PCT International Publication No. WO2002/14490
PCT International Publication No. WO2008/024372
PCT International Publication No. WO2013/086219
PCT International Application No. PCT/US2016/059078, filed October 27, 2016,
PCT International Publication No. WO2002/14490
PCT International Publication No. WO2008/79895
U.S Patent No. 5,264,366
U.S. Patent No. 5,955,310
U.S. Patent No. 5,958,739.
U.S. Patent No. 6,255,076
U.S. Patent No. 8,361,755
U.S. Patent Publication No. 2010/0028944
Anagnostopoulos and Spizizen, J. Bacteriol. 81: 741-746, 1961*.*
Ashikaga et al., "Natural genetic competence in Bacillus subtilis natto OK2", J. Bacteriology 182(9): 2411-2415, 2000*.*
Avery, Trends Microbiol. 13: 459-462, 2005*.*
Berka et al., Mol. Microbiol. 43: 1331-1345, 2002*.*
Bolhuis et al., "Signal peptide peptidase and ClpP-like proteins of Bacillus subtilis required for efficient translocation and processing of secretory proteins", J. Biol. Chem., 274(35):24585-24592, 1999*.*
Brigidi et al., FEMS Microbiol. Lett., 55: 135-138, 1990*.*
Brode et al., "Subtilisin BPN' variants: increased hydrolytic activity on surface-bound substrates via decreased surface activity", Biochemistry, 35(10):3162-3169, 1996*.*
Caspers et al., "Improvement of Sec-dependent secretion of a heterologous model protein in Bacillus subtilis by saturation mutagenesis of the N-domain of the AmyE signal peptide", AppI. Microbiol. Biotechnol., 86(6):1877-1885, 2010*.*
Chen et al., "Combinatorial Sec pathway analysis for improved heterologous protein secretion in Bacillus subtilis: identification of bottlenecks by systematic gene overexpression", Microb. Cell Fact. 14(92), 0282-0289, 2015*.*
D'Souza et al., Proc. Natl. Acad. Sci., USA, 91:9397-9401, 1994.
DeBoer et al., Proceedings of the National Academy of Sciences USA 80:21-25, 1983*.*
Dubnau, Annual Rev. Microbiol. 53: 217-244, 1999*.*
Earl et al., "Ecology and genomics of Bacillus subtilis", Trends in Microbiology.,16(6):269-275, 2008*.*
Fabret et al., "A New Mutation Delivery System for Genome-Scale Approaches in Bacillus subtilis", Molecular Miocrobiology, 46(1):25-36, 2002*.*
Ferrari et al., "Genetics," in Hardwood et al, (eds.), Bacillus, Plenum Publishing Corp., pages 57-72, 1989*.*
Frisby and Zuber, "Analysis of the upstream activating sequence and site of carbon and nitrogen source repression in the promoter of an early-induced sporulation gene of Bacillus subtilis", J. Bacteriology, 173(23): 7557-7556, 1991*.*
Goosens et al., "The Tat system of Gram-positive bacteria", Biochim. Biophys. Acta., 1843(8): 1698-1706, 2014*.*
Hamoen et al., "A small gene, designated corns, located within the coding region of the fourth amino acid-activation domain of srfA, is required for competence development in Bacillus subtilis", Mol. Microbiol., 15(1):55-62, 1995*.*
Hamoen et al., "Controlling competence in Bacillus subtilis: shared used of regulators", Microbiology, 149:9-17, 2003*.*
Hamoen et al., Genes Dev. 12:1539- 1550, 1998*.*
Harwood and Cranenburgh, "Bacillus protein secretion: an unfolding story" Trends in Microbiology, 16(2):73-79, 2007*.*
Harwood et al., Molecular Biological Methods For Bacillus, John Wiley, 1990.
Heinrich et al., "The Bacillus subtilis ABC transporter EcsAB influences intramembrane proteolysis through RasP", Microbiology, 154: 1989-1997, 2008*.*
Hoffmann et al., "Facilitation of direct conditional knockout of essential genes in Bacillus licheniformis DSM13 by comparative genetic analysis and manipulation of genetic competence", Appl. Environ. Microbiol., 76(15): 5046-5057, 2010*.*
Hue et al., Journal of Bacteriology 177: 3465-3471, 1995.
Jakobs et al., Appl. Microbiol. Biotechnol., 99: 2255-2266, 2015*.*
Jensen et al., "Cell-associated degradation affects the yield of secreted engineered and heterologous proteins in the Bacillus subtilis expression system" Microbiology, 146 (Pt 10:2583-2594, 2000*.*
Kang et al., "Molecular engineering of secretory machinery components for high-level secretion of proteins in Bacillus species", J. Ind. Microbiol. Biotechnol., 41(11):1599-1607, 2014*.*
Kim et al., Gene 181: 71-76, 1996*.*
Kontinen and Sarvas, "The PrsA lipoprotein is essential for protein secretion in Bacillus subtilis and sets a limit for high-level secretion", Mol. Microbiol. 8(4):727-737, 1993*.*
Lapidus et al., "Co-linear scaffold of the Bacillus licheniformis and Bacillus subtilis genomes and its use to compare their competence genes", FEMS Microbiol. Lett. 209(1): 20-30, 2002*.*
Nakano and Zuber, "The primary role of comA in establishment of the competent state in Bacillus subtilis is to activate expression of srfA", J. Bacteriology, 173(22):7269-7274, 1991*.*
Olempska-Beer et al., "Food-processing enzymes from recombinant microorganisms--a review"' Regul. Toxicol. Pharmacol., 45(2):144-158, 2006*.*
Raul et al., "Production and partial purification of alpha amylase from Bacillus subtilis (MTCC 121) using solid state fermentation", Biochemistry Research International, 2014.
Roggiani and Dubnau, "ComA, a phosphorylated response regulator protein of Bacillus subtilis, binds to the promoter region of srfA", J. Bacteriology 175(10): 3182-3187, 1993*.*
Rygus and Hillen, J. Bacteriol. 174: 3049-3055, 1992*.*
Saito et al., "Post-liberation cleavage of signal peptidase is catalyzed by the site-21 protease (S2P) in bacteria", PNAS, 108(33): 13740-13745, 2011*.*
Sambrook et al., MOLECULAR CLONING: A LABORATORY MANUAL, 2nd ed., Cold Spring Harbor, 1989*, and* 3rd ed., 2001*.*
Sambrook, Fritsch, and Maniatus, Molecular Cloning, A Laboratory Manual, 2d edition, Cold Spring Harbor, N.Y., 1989*.*
Thome and Skill, J. Bacteriol. 91: 1012-1020, 1966*.*
Tjalsma et al., "Bacillus subtilis contains four closely related type I signal peptidases with overlapping substrate specificities", J. Biol. Chem., 272(41):25983-25992, 1997*.*
Tjalsma et al., "Proteomics of protein secretion by Bacillus subtilis: Separating the 'secrets' of the secretome", Microbiology and Molecular Biology Reviews, 68(2):207-233, 2004*.*
Turgay et al., EMBO J. 17: 6730-6738. 1998*.*
Van Dijl and Hecker, "Bacillus subtilis: from soil bacterium to super-secreting cell factory", Microbial Cell Factories, 12(3). 2013*.*
Van Dijl et al., "Non-functional expression of Escherichia coli signal peptidase I in Bacillus subtilis", Journal of General Microbiology, 137:2073-83, 1991*.*
van Solingen, Extremophiles, Oct;5(5):333-341, 2001*.*
Villa-Kamaroff et al., Proceedings of the National Academy of Sciences USA, 75:3727-3731, 1978*.*
Vogtentanz et al., "A Bacillus subtilis fusion protein system to produce soybean Bowman-Birk protease inhibitor", Protein Expr Purif. 55(1):40-52, 2007*.*
Voskuil et al., Molecular Microbiology 17: 271-279, 1995*.*
Wang et al., "Expression and secretion of human atrial natriuretic alpha-factor in Bacillus subtilis using the subtilisin signal peptide", Gene, 69(1):39-47, 1988*.*
Westers et al., "Bacillus subtilis as cell factory for pharmaceutical proteins: a biotechnological approach to optimize the host organism", Biochimica et Biophysica Acta., 1694:299-310, 2004*.*

## Claims

1. A method for enhancing transformation efficiency of a Bacillus licheniformis host cell comprising:
(a) introducing into a parental Bacillus licheniformis host cell at least one copy of an exogenous nucleic acid construct comprising a promoter region operably linked to a polynucleotide encoding a ComS1 polypeptide, wherein the ComS1 polypeptide comprises an amino acid sequence comprising at least 90% sequence identity to SEQ ID NO: 2, and
(b) isolating a daughter Bacillus licheniformis host cell comprising the polynucleotide encoding the ComS1 polypeptide.

2. A method for obtaining a Bacillus licheniformis transformant comprising:
(a) providing a Bacillus licheniformis host cell in which transformation efficiency has been enhanced by introducing into a parental Bacillus licheniformis host cell at least one copy of a nucleic acid construct comprising a promoter region operably linked to a polynucleotide encoding a ComS1 polypeptide, wherein the ComS1 polypeptide comprises an amino acid sequence comprising at least 90% sequence identity to SEQ ID NO: 2,
(b) transforming an exogenous polynucleotide into a Bacillus licheniformis host cell provided in step (a), and
(c) isolating a transformant of the Bacillus licheniformis host cell comprising the exogenously introduced polynucleotide.

3. The method of claim 1 or claim 2, wherein said parental Bacillus licheniformis host cell does not natively comprise a ComS1 polypeptide comprising an amino acid sequence comprising at least 90% sequence identity to SEQ ID NO: 2.

4. A competent Bacillus licheniformis host cell comprising at least one copy of an exogenously introduced nucleic acid construct comprising a promoter region operably linked to a polynucleotide sequence encoding a ComS1 polypeptide, wherein the ComS1 polypeptide comprises at least 90% sequence identity to SEQ ID NO: 2.

5. A transformed Bacillus licheniformis host cell according to claim 4, wherein the Bacillus licheniformis host cell is transformed with a polynucleotide encoding a protein of interest (POI).

6. The method of claims 1-3 or Bacillus licheniformis host cell of claims 4-5, wherein the polynucleotide encoding the ComS1 polypeptide comprises at least 90% sequence identity to nucleotide sequence of SEQ ID NO: 1 or SEQ ID NO: 21.

7. The method of claims 1-3 or Bacillus licheniformis host cell of claims 4-5, wherein the host cell further comprises at least one copy of an exogenously introduced nucleic acid construct comprising a promoter region operably linked to a polynucleotide encoding a ComK polypeptide, optionally wherein the ComK polypeptide comprises an amino acid sequence comprising 90% sequence identity to SEQ ID NO: 17 or SEQ ID NO: 18.

8. A method for enhancing transformation efficiency of a Bacillus licheniformis (daughter) host cell derived from a parental Bacillus licheniformis host cell, wherein the parental Bacillus licheniformis host cell comprises a native gene encoding a ComS2 polypeptide comprising 90% sequence identity to the ComS2 polypeptide of SEQ ID NO: 4, the method comprising:
(a) identifying a parental Bacillus licheniformis host cell which comprises a native gene encoding a ComS2 polypeptide comprising 90% sequence identity to the ComS2 polypeptide of SEQ ID NO: 4,
(b) deleting or disrupting the native ComS2 gene identified in the parental Bacillus licheniformis host cell of step (a), wherein the deletion or disruption of the native ComS2 gene enhances transformation efficiency of the Bacillus licheniformis daughter host cell derived therefrom, and
(c) isolating the daughter Bacillus licheniformis host cell of step (b).

9. The method of claim 8, wherein the Bacillus licheniformis daughter host cell isolated in step (c) is further modified by introducing at least one copy of an exogenously introduced nucleic acid construct comprising a promoter region operably linked to a polynucleotide sequence encoding a ComS1 polypeptide, wherein the ComS1 polypeptide comprises at least 90% sequence identity to SEQ ID NO: 2.

10. The method of claim 1 or 8, wherein the daughter Bacillus licheniformis host cell is transformed with an exogenously introduced polynucleotide encoding a protein of interest (POI).

11. The method of claim 9, wherein the polynucleotide encoding the ComS1 polypeptide comprises at least 90% sequence identity to nucleotide sequence of SEQ ID NO: 1 or SEQ ID NO: 21.

12. The method of claim 11, wherein the host cell further comprises at least one copy of an exogenously introduced nucleic acid construct comprising a promoter region operably linked to a polynucleotide encoding a ComK polypeptide, optionally wherein the ComK polypeptide comprises an amino acid sequence comprising 90% sequence identity to SEQ ID NO: 17 or SEQ ID NO: 18.

13. A competent Bacillus host cell obtained from the method of claim 8.

14. The transformed host cell of claim 5, or the method of claim 10, wherein the POI is selected from the group consisting of acetyl esterases, aminopeptidases, amylases, arabinases, arabinofuranosidases, carbonic anhydrases, carboxypeptidases, catalases, cellulases, chitinases, chymosins, cutinases, deoxyribonucleases, epimerases, esterases, agalactosidases, 13-galactosidases, a- glucanases, glucan lysases, endo-13-glucanases, glucoamylases, glucose oxidases, a- glucosidases, 13-glucosidases, glucuronidases, glycosyl hydrolases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, perhydrolases, polyol oxidases, peroxidases, phenoloxidases, phytases, polygalacturonases, proteases, peptidases, rhamno-galacturonases, ribonucleases, transferases, transport proteins, transglutaminases, xylanases, hexose oxidases, and combinations thereof.

15. A method for producing a protein of interest, comprising recovering the protein of interest from the transformed Bacillus licheniformis cell of claim 5.

## Patentansprüche

1. Verfahren zur Steigerung der Transformationseffizienz einer Bacillus licheniformis-Wirtszelle, umfassend:
(a) Einführen, in eine elterliche Bacillus licheniformis-Wirtszelle, von mindestens einer Kopie eines exogenen Nukleinsäurekonstrukts, umfassend eine Promotorregion, die operativ mit einem Polynukleotid gekoppelt ist, das ein ComSl-Polypeptid codiert, wobei das ComSl-Polypeptid eine Aminosäuresequenz umfasst, umfassend mindestens 90% Sequenzidentität mit SEQ ID NO: 2, und
(b) Isolieren einer Tochter-Bacillus licheniformis-Wirtszelle, umfassend das Polynukleotid, das das ComSl-Polypeptid codiert.

2. Verfahren zur Gewinnung eines Bacillus licheniformis-Transformanten, umfassend:
(a) Bereitstellen einer Bacillus licheniformis-Wirtszelle, in der die Transformationseffizienz durch Einführen mindestens einer Kopie eines Nukleinsäurekonstrukts in eine elterliche Bacillus licheniformis-Wirtszelle gesteigert wurde, wobei das Nukleinsäurekonstrukt eine Promotorregion umfasst, die operativ mit einem Polynukleotid gekoppelt ist, das ein ComSl-Polypeptid codiert, wobei das ComSl-Polypeptid eine Aminosäuresequenz umfasst, umfassend mindestens 90 % Sequenzidentität mit SEQ ID NO: 2,
(b) Transformieren eines exogenen Polynukleotids in eine in Schritt (a) bereitgestellte Bacillus licheniformis-Wirtszelle, und
(c) Isolieren einer Transformanten der Bacillus licheniformis-Wirtszelle, umfassend das exogen eingeführte Polynukleotid.

3. Verfahren nach Anspruch 1 oder Anspruch 2, wobei die elterliche Bacillus licheniformis-Wirtszelle nicht nativ ein ComSl-Polypeptid umfasst, umfassend eine Aminosäuresequenz, umfassend mindestens 90% Sequenzidentität mit SEQ ID NO: 2.

4. Kompetente Bacillus licheniformis-Wirtszelle, umfassend mindestens eine Kopie eines exogen eingeführten Nukleinsäurekonstrukts, umfassend eine Promotorregion, die operativ mit einer Polynukleotidsequenz gekoppelt ist, die ein ComSl-Polypeptid codiert, wobei das ComSl-Polypeptid mindestens 90 % Sequenzidentität mit SEQ ID NO: 2 umfasst.

5. Transformierte Bacillus licheniformis-Wirtszelle nach Anspruch 4, wobei die Bacillus licheniformis-Wirtszelle mit einem Polynukleotid transformiert ist, das ein Protein von Interesse (POI) codiert.

6. Verfahren nach Anspruch 1 bis 3 oder Bacillus licheniformis-Wirtszelle nach Anspruch 4 bis 5, wobei das Polynukleotid, das das ComSl-Polypeptid codiert, mindestens 90% Sequenzidentität mit der Nukleotidsequenz von SEQ ID NO: 1 oder SEQ ID NO: 21 umfasst.

7. Verfahren nach Anspruch 1 bis 3 oder Bacillus licheniformis-Wirtszelle nach Anspruch 4 bis 5, wobei die Wirtszelle ferner mindestens eine Kopie eines exogen eingeführten Nukleinsäurekonstrukts umfasst, umfassend eine Promotorregion, die operativ mit einem Polynukleotid gekoppelt ist, das ein ComK-Polypeptid codiert, wobei das ComK-Polypeptid gegebenenfalls eine Aminosäuresequenz umfasst, umfassend 90% Sequenzidentität mit SEQ ID NO: 17 oder SEQ ID NO: 18.

8. Verfahren zur Verstärkung der Transformationseffizienz einer Bacillus licheniformis (Tochter)-Wirtszelle, die von einer elterlichen Bacillus licheniformis-Wirtszelle stammt, wobei die elterliche Bacillus licheniformis-Wirtszelle ein natives Gen umfasst, das ein ComS2-Polypeptid kodiert, umfassend 90 % Sequenzidentität mit dem ComS2-Polypeptid der SEQ ID NO: 4, wobei das Verfahren umfasst:
(a) Identifizierung einer elterlichen Bacillus licheniformis-Wirtszelle, die ein natives Gen umfasst, das ein ComS2-Polypeptid codiert, umfassend 90 % Sequenzidentität mit dem ComS2-Polypeptid von SEQ ID NO: 4,
(b) Deletion oder Disruption des nativen ComS2-Gens, das in der elterlichen Bacillus licheniformis-Wirtszelle von Schritt (a) identifiziert wurde, wobei die Deletion oder Disruption des nativen ComS2-Gens die Transformationseffizienz der daraus stammenden Bacillus licheniformis-Tochterwirtszelle steigert, und
(c) Isolierung der Tochter-Bacillus licheniformis-Wirtszelle aus Schritt (b).

9. Verfahren nach Anspruch 8, wobei die in Schritt (c) isolierte Bacillus licheniformis-Tochterwirtszelle weiter modifiziert wird, indem mindestens eine Kopie eines exogen eingeführten Nukleinsäurekonstrukts eingeführt wird, umfassend eine Promotorregion, die operativ mit einer Polynukleotidsequenz gekoppelt ist, die ein ComSl-Polypeptid codiert, wobei das ComSl-Polypeptid mindestens 90% Sequenzidentität mit SEQ ID NO: 2 umfasst.

10. Verfahren nach Anspruch 1 oder 8, wobei die TochterWirtszelle von Bacillus licheniformis mit einem exogen eingeführten Polynukleotid transformiert wird, das ein Protein von Interesse (POI) codiert.

11. Verfahren nach Anspruch 9, wobei das Polynukleotid, das das ComSl-Polypeptid codiert, mindestens 90% Sequenzidentität mit der Nukleotidsequenz von SEQ ID NO: 1 oder SEQ ID NO: 21 umfasst.

12. Verfahren nach Anspruch 11, wobei die Wirtszelle ferner mindestens eine Kopie eines exogen eingeführten Nukleinsäurekonstrukts umfasst, das eine Promotorregion umfasst, die funktionsfähig mit einem Polynukleotid verbunden ist, das für ein ComK-Polypeptid kodiert, wobei das ComK-Polypeptid gegebenenfalls eine Aminosäuresequenz umfasst, die 90 % Sequenzidentität mit SEQ ID NO: 17 oder SEQ ID NO: 18 aufweist.

13. Kompetente Bacillus-Wirtszelle, erhalten durch das Verfahren nach Anspruch 8.

14. Transformierte Wirtszelle nach Anspruch 5 oder Verfahren nach Anspruch 10, wobei das POI ausgewählt ist aus der Gruppe bestehend aus Acetylesterasen, Aminopeptidasen, Amylasen, Arabinasen, Arabinofuranosidasen, Carbonatanhydrasen, Carboxypeptidasen, Katalasen, Cellulasen, Chitinasen, Chymosine, Cutinasen, Desoxyribonukleasen, Epimerasen, Esterasen, Agalactosidasen, 13-Galactosidasen, α-Glucanasen, Glucan-Lysasen, Endo-13-Glucanasen, Glucoamylasen, Glucoseoxidasen, α-Glucosidasen, 13-Glucosidasen, Glucuronidasen, Glycosylhydrolasen, Hemicellulasen, Hexoseoxidasen, Hydrolasen, Invertasen, Isomerasen, Laccasen, Lipasen, Lyasen, Mannosidasen, Oxidasen, Oxidoreduktasen, Pektatlyasen, Pektinacetylesterasen, Pektindepolymerasen, Pektinmethylesterasen, pektinolytischen Enzymen, Perhydrolasen, Polyoloxidasen, Peroxidasen, Phenoloxidasen, Phytasen, Polygalacturonasen, Proteasen, Peptidasen, Rhamno-Galacturonasen, Ribonukleasen, Transferasen, Transportproteine, Transglutaminasen, Xylanasen, Hexoseoxidasen und Kombinationen davon.

15. Verfahren zur Herstellung eines Proteins von Interesse, umfassend die Gewinnung des Proteins von Interesse aus der transformierten Bacillus licheniformis-Zelle nach Anspruch 5.

## Revendications

1. Procédé pour l'augmentation de l'efficacité de transformation d'une cellule hôte de Bacillus licheniformis comprenant :
(a) l'introduction dans une cellule hôte de Bacillus licheniformis parentérale d'au moins une copie d'une construction d'acide nucléique exogène comprenant une région de promoteur liée de manière fonctionnelle à un polynucléotide codant pour un polypeptide ComSl, le polypeptide ComS1 comprenant une séquence d'acides aminés comprenant au moins 90 % d'identité de séquence avec la SEQ ID NO: 2, et
(b) l'isolement d'une cellule hôte de Bacillus licheniformis fille comprenant le polynucléotide codant pour le polypeptide ComS1.

2. Procédé pour l'obtention d'un transformant de Bacillus licheniformis comprenant :
(a) la fourniture d'une cellule hôte de Bacillus licheniformis dans laquelle une efficacité de transformation a été augmentée en introduisant dans une cellule hôte de Bacillus licheniformis parentérale au moins une copie d'une construction d'acide nucléique comprenant une région de promoteur liée de manière fonctionnelle à un polynucléotide codant pour un polypeptide ComSl, le polypeptide ComS1 comprenant une séquence d'acides aminés comprenant au moins 90 % d'identité de séquence avec la SEQ ID NO: 2,
(b) la transformation d'un polynucléotide exogène dans une cellule hôte de Bacillus licheniformis fournie dans l'étape (a), et
(c) l'isolement d'un transformant de la cellule hôte de Bacillus licheniformis comprenant le polynucléotide introduit de manière exogène.

3. Procédé selon la revendication 1 ou la revendication 2, ladite cellule hôte de Bacillus licheniformis parentérale ne comprenant pas de manière native un polypeptide ComS1 comprenant une séquence d'acides aminés comprenant au moins 90 % d'identité de séquence avec la SEQ ID NO: 2.

4. Cellule hôte de Bacillus licheniformis compétente comprenant au moins une copie d'une construction d'acide nucléique introduite de manière exogène comprenant une région de promoteur liée de manière fonctionnelle à une séquence polynucléotidique codant pour un polypeptide ComSl, le polypeptide ComS1 comprenant au moins 90 % d'identité de séquence avec la SEQ ID NO: 2.

5. Cellule hôte de Bacillus licheniformis transformée selon la revendication 4, la cellule hôte de Bacillus licheniformis étant transformée avec un polynucléotide codant pour une protéine d'intérêt (POI).

6. Procédé selon les revendications 1 à 3 ou cellule hôte de Bacillus licheniformis selon les revendications 4 et 5, le polynucléotide codant pour le polypeptide ComS1 comprenant au moins 90 % d'identité de séquence avec la SEQ ID NO: 1 ou la SEQ ID NO: 21.

7. Procédé selon les revendications 1 à 3 ou cellule hôte de Bacillus licheniformis selon les revendications 4 et 5, la cellule hôte comprenant en outre au moins une copie d'une construction d'acide nucléique introduite de manière exogène comprenant une région de promoteur liée de manière fonctionnelle à un polynucléotide codant pour un polypeptide ComK, éventuellement, le polypeptide ComK comprenant une séquence d'acides aminés comprenant 90 % d'identité de séquence avec la SEQ ID NO: 17 ou la SEQ ID NO: 18.

8. Procédé pour l'augmentation de l'efficacité de transformation d'une cellule hôte de Bacillus licheniformis (fille) issue d'une cellule hôte de Bacillus licheniformis parentérale, la cellule hôte de Bacillus licheniformis parentérale comprenant un gène natif codant pour un polypeptide ComS2 comprenant 90 % d'identité de séquence avec le polypeptide ComS2 de la SEQ ID NO: 4, le procédé comprenant :
(a) l'identification d'une cellule hôte de Bacillus licheniformis parentérale qui comprend un gène natif codant pour un polypeptide ComS2 comprenant 90 % d'identité de séquence avec le polypeptide ComS2 de la SEQ ID NO: 4,
(b) la délétion ou la perturbation du gène ComS2 natif identifié dans la cellule hôte de Bacillus licheniformis parentérale de l'étape (a), la délétion ou perturbation du gène ComS2 natif augmentant l'efficacité de transformation de la cellule hôte fille de Bacillus licheniformis issue de celle-ci, et
(c) l'isolement de la cellule hôte de Bacillus licheniformis fille de l'étape (b).

9. Procédé selon la revendication 8, la cellule hôte fille de Bacillus licheniformis isolée dans l'étape (c) étant en outre modifiée en introduisant au moins une copie d'une construction d'acide nucléique introduite de manière exogène comprenant une région de promoteur liée de manière fonctionnelle à une séquence de polynucléotides codant pour un polypeptide ComSl, le polypeptide ComS1 comprenant au moins 90 % d'identité de séquence avec la SEQ ID NO: 2.

10. Procédé selon la revendication 1 ou 8, la cellule hôte de Bacillus licheniformis fille étant transformée par un polynucléotide introduit de manière exogène codant pour une protéine d'intérêt (POI) .

11. Procédé selon la revendication 9, le polynucléotide codant pour le polypeptide ComS1 comprenant au moins 90 % d'identité de séquence avec la séquence de nucléotides de la SEQ ID NO: de 1 ou de la SEQ ID NO: 21.

12. Procédé selon la revendication 11, la cellule hôte comprenant en outre au moins une copie d'une construction d'acide nucléique introduite de manière exogène comprenant une région de promoteur liée de manière fonctionnelle à un polynucléotide codant pour un polypeptide ComK, éventuellement, le polypeptide ComK comprenant une séquence d'acides aminés comprenant 90 % d'identité de séquence avec la SEQ ID NO: 17 ou la SEQ ID NO: 18.

13. Cellule hôte de Bacillus compétente obtenue par le procédé selon la revendication 8.

14. Cellule hôte transformée selon la revendication 5, ou procédé selon la revendication 10, la POI étant choisie dans le groupe constitué par les acétylestérases, les aminopeptidases, les amylases, les arabinases, les arabinofuranosidases, les carbonique anhydrases, les carboxypeptidases, les catalases, les cellulases, les chitinases, les chymosines, les cutinases, les désoxyribonucléases, les épimérases, les estérases, les agalactosidases, les 13-galactosidases, les α-glucanases, les glucane lysases, les endo-13-glucanases, les glucoamylases, les glucose oxydases, les a-glucosidases, les 13-glucosidases, les glucuronidases, les glycosyl-hydrolases, les hémicellulases, les hexose oxydases, les hydrolases, les invertases, les isomérases, les laccases, les lipases, les lyases, les mannosidases, les oxydases, les oxydoréductases, les pectate lyases, les pectine acétyl-estérases, les pectine dépolymérases, les pectine méthyl-estérases, les enzymes pectinolytiques, les perhydrolases, les polyol oxydases, les peroxydases, les phénoloxydases, les phytases, les polygalacturonases, les protéases, les peptidases, les rhamno-galacturonases, les ribonucléases, les transférases, les protéines de transport, les transglutaminases, les xylanases, les hexose oxydases et des combinaisons correspondantes.

15. Procédé pour la production d'une protéine d'intérêt, comprenant la récupération de la protéine d'intérêt à partir de la cellule de Bacillus licheniformis transformée selon la revendication 5.
